# FASCICULE DE BREVET EUROPEEN

(11) **EP 2 748 196 B1**
(45) Date de publication et mention de la délivrance du brevet: **05.10.2016**
(21) Numéro de dépôt: 12751318.2
(22) Date de dépôt: 22.08.2012
(51) Int. Cl.: C07K 16/28, A61K 39/395, A61P 9/00

(54) **NANOCORPS ANTI-VCAM-1**
ANTI-VCAM-1-NANOKÖRPER
ANTI-VCAM-1 NANOBODIES

(30) Priorité: 23.08.2011 FR 1157478
(43) Date de publication de la demande: 02.07.2014
(73) Titulaire: Université Joseph Fourier - Grenoble, 38041 Grenoble Cedex 9 (FR)
(72) Inventeur: GHEZZI, Catherine, F-38000 Grenoble (FR); FAGRET, Daniel, F-38000 Grenoble (FR); BROISAT, Alexis, F-38000 Grenoble (FR); DEVOOGDT, Nick, B-1980 Zemst (BE); LAHOUTTE, Tony, B-1083 Ganshoren (BE); MUYDERMANS, Serge, B-1560 Hoeilaart (BE)
(74) Mandataire: Lavoix
(86) Numéro de dépôt international: PCT/EP2012/066348
(87) Numéro de publication internationale: WO 2013/026878

(56) Documents cités:
- EP-A1- 2 206 726
- EP-A2- 1 520 588
- FR-A1- 2 876 033
- FR-A1- 2 914 304
- ILSE VANEYCKEN ET AL: "Immuno-imaging using nanobodies", CURRENT OPINION IN BIOTECHNOLOGY, vol. 22, no. 6, décembre 2011 (2011-12), pages 877-881, XP055021086, ISSN: 0958-1669, DOI: 10.1016/j.copbio.2011.06.009
- BROISAT A ET AL: "Molecular imaging of vascular cell adhesion molecule-1 expression in experimental atherosclerotic plaques with radiolabelled B2702-p", EUROPEAN JOURNAL OF NUCLEAR MEDICINE AND MOLECULAR IMAGING, SPRINGER, BERLIN, DE, vol. 34, no. 6, 12 janvier 2007 (2007-01-12), pages 830-840, XP019514008, ISSN: 1619-7089, DOI: 10.1007/S00259-006-0310-4 cité dans la demande
- DIMASTROMATTEO J ET AL: "A030 In vivo molecular imaging of vascular cell adhesion molecule-1 expression in atherosclerotic plaques", ARCHIVES OF CARDIOVASCULAR DISEASE, ELSEVIER, AMSTERDAM, NL, vol. 102, 1 mars 2009 (2009-03-01), page S17, XP026054546, ISSN: 1875-2136, DOI: 10.1016/S1875-2136(09)72163-1 [extrait le 2009-03-01]
- L. M. RIOU ET AL: "Pre-Clinical and Clinical Evaluation of Nuclear Tracers for the Molecular Imaging of Vulnerable Atherosclerosis: An Overview", CURRENT MEDICINAL CHEMISTRY, vol. 16, no. 12, 1 avril 2009 (2009-04-01) , pages 1499-1511, XP055021246, ISSN: 0929-8673, DOI: 10.2174/092986709787909596 cité dans la demande
- B. A. KAUFMANN ET AL: "Molecular Imaging of Inflammation in Atherosclerosis With Targeted Ultrasound Detection of Vascular Cell Adhesion Molecule-1", CIRCULATION, vol. 116, no. 3, 17 juillet 2007 (2007-07-17), pages 276-284, XP055020746, ISSN: 0009-7322, DOI: 10.1161/CIRCULATIONAHA.106.684738
- C. Z. BEHM ET AL: "Molecular Imaging of Endothelial Vascular Cell Adhesion Molecule-1 Expression and Inflammatory Cell Recruitment During Vasculogenesis and Ischemia-Mediated Arteriogenesis", CIRCULATION, vol. 117, no. 22, 1 juin 2008 (2008-06-01) , pages 2902-2911, XP055020750, ISSN: 0009-7322, DOI: 10.1161/CIRCULATIONAHA.107.744037
- MATTHIAS NAHRENDORF ET AL: "Noninvasive vascular cell adhesion molecule-1 imaging identifies inflammatory activation of cells in atherosclerosis", CIRCULATION, LIPPINCOTT WILLIAMS & WILKINS, US, vol. 114, no. 14, 1 octobre 2006 (2006-10-01), pages 1504-1511, XP002639504, ISSN: 0009-7322, DOI: 10.1161/CIRCULATIONAHA.106.646380
- KELLY K A ET AL: "Detection of Vascular Adhesion Molecule-1 Expression Using a Neovel Multimodal Nanoparticle", CIRCULATION RESEARCH, GRUNE AND STRATTON, BALTIMORE, US, vol. 96, 18 février 2005 (2005-02-18), pages 327-336, XP002456858, ISSN: 0009-7330, DOI: 10.1161/01.RES.0000155722.17881.DD
- BROISAT A ET AL: "Imagerie moleculaire de la plaque d'atherome vulnerable. Evaluation preclinique et clinique de traceurs radioactifs", MEDECINE NUCLEAIRE, ELSEVIER, PARIS, FR, vol. 33, no. 3, 1 mars 2009 (2009-03-01), pages 128-136, XP025968615, ISSN: 0928-1258, DOI: 10.1016/J.MEDNUC.2009.01.004 [extrait le 2009-02-25]
- DE GROEVE KURT ET AL: "Nanobodies as tools for in vivo imaging of specific immune cell types", JOURNAL OF NUCLEAR MEDICINE, SOCIETY OF NUCLEAR MEDICINE, RESTON, VA, US, vol. 51, no. 5, 1 mai 2010 (2010-05-01), pages 782-789, XP009152829, ISSN: 0161-5505
- SOPHIE HERNOT ET AL: "Nanobody-coupled microbubbles as novel molecular tracer", JOURNAL OF CONTROLLED RELEASE, vol. 158, no. 2, 1 mars 2012 (2012-03-01), pages 346-353, XP055020683, ISSN: 0168-3659, DOI: 10.1016/j.jconrel.2011.12.007
- A. BROISAT ET AL: "Nanobodies Targeting Mouse/Human VCAM1 for the Nuclear Imaging of Atherosclerotic Lesions", CIRCULATION RESEARCH, vol. 110, no. 7, 30 mars 2012 (2012-03-30) , pages 927-937, XP55041152, ISSN: 0009-7330, DOI: 10.1161/CIRCRESAHA.112.265140

## Description

La présente invention concerne des nanocorps anti-VCAM-1 permettant la détection *in vivo* de plaques d'athérome.

Les maladies cardiovasculaires représentent la première cause mondiale de mortalité et la maladie coronaire est responsable à elle seule de plus de la moitié de ces décès. La survenue d'un accident coronaire est due, dans l'immense majorité des cas, à la rupture d'une plaque d'athérome coronaire vulnérable. La coronographie, technique de référence actuelle pour le diagnostic de la maladie coronaire, ne permet pas l'identification des plaques non-sténosantes.

L'imagerie nucléaire présente à l'évidence des avantages significatifs pour l'imagerie moléculaire de la plaque d'athérome vulnérable. De nombreux traceurs, de natures chimiques variées, parmi lesquels des lipoprotéines, des peptides, des oligopeptides, des anticorps, des sucres, des nucléotides antisens et des nanoparticules, ont été évalués expérimentalement pour l'imagerie moléculaire (Riou et al. (2009) Curr. Med. Chem. 16:1499-1511). Les principales cibles évaluées ont été les LDLs oxydées et leurs récepteurs, le phénomène inflammatoire via l'imagerie cellulaire des macrophages, ou l'imagerie de récepteurs ou d'enzymes exprimés par ce type cellulaire, le phénomène apoptotique et le phénomène néoangiogénique. Parmi les traceurs ciblant le processus inflammatoire, ^{99m}Tc-MCP-1 pour l'imagerie nucléaire en mode SPECT (Single Photon Emission Computed Tomography) et le [¹⁸F]-FDG pour la modalité PET (Positron Emission Tomography) ont permis l'imagerie *in vivo* non invasive de l'accumulation de macrophages dans des lésions athérosclérotiques expérimentales. Sur un plan clinique, [¹⁸F]-FDG et ^{99m}Tc-Annexin A5 ont permis l'imagerie non invasive de l'accumulation de macrophages et des cellules apoptotiques, respectivement, dans les plaques d'athérome carotidiennes de patients symptomatiques. Cependant, aucun de ces radiotraceurs n'est actuellement utilisé en pratique clinique en routine, principalement du fait de leur inaptitude à atteindre des ratios lésions sur bruit de fond suffisants au niveau des lésions coronaires. En effet, l'imagerie nucléaire des plaques vulnérables au niveau des artères coronaires est particulièrement difficile du fait du faible volume des lésions et de leur proximité avec le sang qui contient du traceur circulant non lié. Il n'existe donc pas d'essai clinique à l'heure actuelle montrant la faisabilité de l'imagerie de l'athérome coronaire.

VCAM-1 est une glycoprotéine de la famille des immunoglobulines dont l'expression est induite en condition pro-athérogénique. Son expression est restreinte aux zones de développement des plaques d'athérome et elle perdure pendant la totalité du développement de la plaque vulnérable. Le rôle de VCAM-1 est d'assurer le recrutement des cellules de l'inflammation (lymphocytes et monocytes) vers la plaque. L'expression de VCAM-1 est donc directement corrélée à l'accumulation de macrophages, qui est reconnue comme l'un des critères majeurs dans la définition d'une plaque vulnérable (Naghavi et al. (2003) Circulation 108:1772-1178).

La demande européenne EP 2 206 726 décrit l'utilisation de dérivés du peptide B2702p liant VCAM-1 dans des méthodes d'imagerie médicale *in vivo,* non invasives.

Les présents inventeurs ont précédemment mis au point des radiotraceurs contenant une séquence peptidique capable de se lier à VCAM-1. Ils ont montré sur un modèle de lapin athérosclérotique que la fixation de ce radiotraceur sur les images autoradiographiques *ex vivo* était corrélée aux zones de développement des plaques d'athérome et à l'expression de VCAM-1 (Broisat et al. (2007) Eur. J. Nucl. Med. Mol. Imaging 34:830-840). Cependant du fait de l'activité sanguine circulante de ce traceur, il n'est pas possible de l'utiliser *in vivo* en imagerie médicale.

En effet, d'une manière générale, le rapport signal sur bruit doit être élevé pour réaliser de l'imagerie médicale de qualité. Un radiotraceur idéal est donc caractérisé par une haute affinité et spécificité pour sa cible, une bonne solubilité et stabilité, un radiomarquage efficace, une petite taille ainsi que par une élimination sanguine rapide, de telle sorte que des images à haut niveau de contraste puissent être obtenues rapidement après l'administration du traceur. Ceci est tout particulièrement crucial dans le cas de la plaque d'athérome du fait de sa faible taille et de sa localisation intravasculaire.

La présente invention résulte de la découverte par les inventeurs, que quatre nanocorps, appelés cAbVCAM1-5, cAbVCAM1-3, cAbVCAM1-8 et cAbVCAM1-9, comprenant des séquences de CDRs spécifiques, présentaient de manière surprenante, et contrairement à d'autres nanocorps se liant à VCAM-1, toutes les caractéristiques définies ci-dessus et constituaient donc des radiotraceurs efficaces pour l'imagerie médicale non invasive des plaques d'athérome, en particulier des plaques d'athérome coronaires.

La présente invention a donc pour objet un nanocorps dirigé contre VCAM-1 comprenant :
a) les séquences d'acides aminés (i) YTNSIMYMA (SEQ ID NO: 1) comme CDR1, (ii) AIRFPDDS (SEQ ID NO: 2) comme CDR2 et (iii) RSSPYSFAWNDPSNYNY (SEQ ID NO: 3) comme CDR3; ou
b) les séquences d'acides aminés (i) FTYSSYYMS (SEQ ID NO: 4) comme CDR1, (ii) GINVDGSN (SEQ ID NO: 5) comme CDR2 et (iii) GSGRDSYDCYSGSWCP (SEQ ID NO: 6) comme CDR3; ou
c) les séquences d'acides aminés (i) FTFSNYYMT (SEQ ID NO: 7) comme CDR1, (ii) RINSDGS (SEQ ID NO: 8) comme CDR2 et (iii) GKSSV (SEQ ID NO: 9) comme CDR3; ou
d) les séquences d'acides aminés (i) FTFSSYYMS (SEQ ID NO: 10) comme CDR1, (ii) GINVDGSN (SEQ ID NO: 11) comme CDR2 et (iii) GSGRDSYDCYSGSWCP (SEQ ID NO: 12) comme CDR3;
ou un variant fonctionnellement conservatif du nanocorps défini en a), b), c) ou d) comprenant une substitution conservative d'un ou deux acides aminés dans respectivement une, deux ou trois des séquences SEQ ID NO: 1, SEQ ID NO: 2 et SEQ ID NO: 3, ou SEQ ID NO: 4, SEQ ID NO: 5 et SEQ ID NO: 6, ou SEQ ID NO: 7, SEQ ID NO: 8 et SEQ ID NO: 9, ou SEQ ID NO: 10, SEQ ID NO: 11 et SEQ ID NO: 12.

La présente invention concerne également le nanocorps tel que défini ci-dessus pour son utilisation comme agent de contraste dans l'imagerie médicale *in vivo,* non invasive, pour son utilisation dans des méthodes de diagnostic ou de pronostic et pour son utilisation comme médicament.

Elle a également pour objet l'utilisation de ce nanocorps pour la détection *in vitro* de VCAM-1 dans un échantillon.

Elle concerne enfin une composition pharmaceutique comprenant ce nanocorps en association avec un véhicule pharmaceutiquement acceptable.

### VCAM-1

Le terme "VCAM-1" ou "vascular cell adhesion molecule 1" désigne une protéine d'adhésion cellulaire dont la transcription est induite dans les cellules endothéliales mais qui est également exprimée par d'autres types cellulaires. VCAM-1 a été découverte et clonée par Osborn *et al.* en 1989 (Osborn et al. (1989) Cell. 59:1203-1211). VCAM-1 interagit avec l'intégrine α4β1, aussi appelée VLA-4 (Very Late Antigen 4), qui est exprimée de manière constitutive par les lymphocytes et les monocytes, notamment. Comme d'autres molécules d'adhésion, telles que ICAM-1, 2 et 3, VCAM-1 est impliquée dans l'adhésion des monocytes à l'endothélium au cours de l'athérosclérose. VCAM-1 interagit également avec l'intégrine α4β7 pour le recrutement de lymphocytes au niveau de l'intestin.

### Nanocorps anti-VCAM-1

Dans le contexte de la présente invention, les termes "anticorps" et "immunoglobuline" ont la même signification et sont utilisés indifféremment. Dans les anticorps conventionnels, les deux chaînes lourdes sont liées l'une à l'autre par des ponts disulfures et chaque chaine lourde est liée à une chaîne légère par un pont disulfure. Il y a deux types de chaîne légère : les chaînes légères lambda (λ) et kappa (κ). Il existe cinq principales classes de chaînes lourdes (ou isotypes) qui déterminent l'activité fonctionnelle d'un anticorps : IgM, IgD, IgG, IgA et IgE. Chaque chaîne contient des domaines de séquence distincts. La chaîne légère comprend deux domaines : un domaine variable (VL) et un domaine constant (CL). La chaîne lourde comprend quatre domaines : un domaine variable (VH) et trois domaines constants (CH1, CH2 et CH3, collectivement nommés CH). Les régions variables des chaînes lourde (VH) et légère (VL) déterminent la reconnaissance de liaison et la spécificité à l'antigène. Les domaines des régions constantes des chaînes légère (CL) et lourde (CH) confèrent des propriétés biologiques importantes telles que l'association des chaînes des anticorps, la sécrétion, la mobilité transplacentaire, la liaison au complément et la liaison aux récepteurs Fc. Le fragment Fv est la partie N-terminale du fragment Fab d'une immunoglobuline consistant en les portions variables d'une chaîne légère et d'une chaîne lourde. La spécificité de l'anticorps réside dans la complémentarité structurale entre le site de combinaison de l'anticorps et le déterminant antigénique. Les sites de combinaison de l'anticorps sont faits de résidus qui proviennent principalement des régions hypervariables ou régions de détermination de la complémentarité (CDRs). Occasionnellement, des résidus provenant des régions non-hypervariables ou régions "charpente" ("framework", FR) peuvent influencer la structure globale du domaine et par conséquent le site de combinaison.

Dans le contexte de l'invention, le terme "CDR" fait référence aux séquences d'acides aminés, qui, ensemble, définissent l'affinité de liaison et la spécificité de la région Fv naturelle d'un site de liaison natif d'une immunoglobuline. Les chaînes lourde et légère d'une immunoglobuline ont chacune trois CDRs, désignés H-CDR1, H-CDR2, H-CDR3 et L-CDR1, L-CDR2, L-CDR3 respectivement. Un site de liaison à l'antigène inclut donc 6 CDRs, comprenant l'ensemble des CDRs d'une région variable d'une chaîne lourde et d'une région variable d'une chaîne légère.

La localisation des CDRs dans la séquence d'un anticorps ou d'un nanocorps peut être déterminée par l'homme du métier en utilisant des techniques précédemment décrites. Typiquement, les CDRs peuvent être identifiés en séquencant l'ADN de l'anticorps ou du nanocorps avec un système approprié, tel que le 3730XL DNA Analyser et ABI PRISM BigDye Terminator cyc, puis en analysant les séquences ainsi obtenues à l'aide de bases de données dédiées telle que le international ImMunoGeneTics database ou IMTG (Lefranc (2003) Dev. Comp. Immunol. 27:55).

Dans le contexte de l'invention, le terme "région charpente", "framework" ou "FR" font référence aux séquences d'acides aminés intercalées entre les CDRs.

Dans le contexte de l'invention, les termes "nanocorps", "nanobody", "VHH", "fragment d'anticorps VHH" et "anticorps à domaine unique" sont utilisés indifféremment et désignent le domaine variable de la chaîne lourde unique d'anticorps du type de ceux trouvés chez les camélidés, qui sont naturellement dépourvus de chaînes légères. En absence de chaîne légère les nanocorps ont chacun trois CDRs, désignés CDR1, CDR2, et CDR3 respectivement. Les nanocorps selon l'invention peuvent en particulier être des nanocorps de chameaux, de dromadaires, de lamas ou d'alpagas. De préférence, les nanocorps selon l'invention sont des nanocorps de dromadaires.

Par "nanocorps dirigé contre VCAM-1", on entend ici un nanocorps capable de se lier de manière sélective à VCAM-1. Préférentiellement, le nanocorps est spécifique de VCAM-1, c'est-à-dire qu'il se lie à VCAM-1 à l'exclusion de toute autre molécule.

Les présents inventeurs ont identifié plus précisément quatre nanocorps dirigés contre VCAM-1 et présentant des caractéristiques additionnelles non attendues que ne possèdent pas d'autres nanocorps anti-VCAM-1. En effet, ces quatre nanocorps, cAbvCAM1-5, cAbVCAM1-3, cAbVCAM1-8, cAbVCAM1-9, présentent une réaction croisée pour VCAM-1 humain et murin; ils ont une très forte affinité pour VCAM-1 murin ou humain, et ils se fixent très fortement dans les plaques d'athérome *in vivo,* sans pour autant induire un bruit de fond dans les organes du patient.

Ces quatre nanocorps ont été séquencés :
- le nanocorps cAbVCAM1-5 présente la séquence d'acides aminés QVQLQESGGGSVQTGGSLRLSCAASGYTNSIMYMAWFRQAPGKKREG VAAIRFPDDSAYYAGSVKGRFTISHDNAKNTVYLQMNNLNPEDTAMYYC AARSSPYSFAWNDPSNYNYWGQGTQVTVSS (SEQ ID NO: 13),
- le nanocorps cAbVCAM1-3 présente la séquence d'acides aminés QVQLQESGGGSVQAGGSLRLSCTASGFTYSSYYMSWVRQAPGKGLE WVSGINVDGSNTYYADSVKGRFTISRDNAKNTLYLQMNSLKSEDTALYY CATGSGRDSYDCYSGSWCPKGQGTQVTVSS (SEQ ID NO: 14),
- le nanocorps cAbVCAM1-8 présente la séquence d'acides aminés QVQLQESGGGLVQPGGSLRLSCAASGFTFSNYYMTWVRRAPGKGLE WVSRINSDGSTLYLPSVKGRFTISRDNAKNTLYLQMNSLKSEDTGWYY CVEGKSSVRGQGTQVTVSS (SEQ ID NO: 15), et
- le nanocorps cAbVCAM1-9 présente la séquence d'acides aminés QVQLQESGGGLVQPGGSLRLSCAASGFTFSSYYMSWVRQAPGKGLE WVSGINVDGSNTYYADSVKGRFTISRDNAKNTLYLQMNSLKSEDTALYY CATGSGRDSYDCYSGSWCPKGQGTQVTVSS (SEQ ID NO: 16).

Les CDRs de ces quatre nanocorps ont été plus spécifiquement séquencés et sont les suivants :
- cAbVCAM1-5
   ∘ CDR1 : YTNSIMYMA (SEQ ID NO: 1)
   ∘ CDR2 : AIRFPDDS (SEQ ID NO: 2)
   ∘ CDR3 : RSSPYSFAWNDPSNYNY (SEQ ID NO: 3)
- cAbVCAM1-3
   ∘ CDR1 : FTYSSYYMS (SEQ ID NO: 4)
   ∘ CDR2 : GINVDGSN (SEQ ID NO: 5)
   ∘ CDR3 : GSGRDSYDCYSGSWCP (SEQ ID NO: 6)
- cAbVCAM1-8
   ∘ CDR1 : FTFSNYYMT (SEQ ID NO: 7)
   ∘ CDR2 : RINSDGS (SEQ ID NO: 8)
   ∘ CDR3 : GKSSV (SEQ ID NO: 9)
- cAbVCAM1-9
   ∘ CDR1 : FTFSSYYMS (SEQ ID NO: 10)
   ∘ CDR2 : GINVDGSN (SEQ ID NO: 11)
   ∘ CDR3 : GSGRDSYDCYSGSWCP (SEQ ID NO: 12)

Comme il est bien connu de l'homme du métier, la combinaison des CDR1, CDR2 et CDR3 suffit pour définir un site de liaison à l'antigène. Par conséquent, un objet de la présente invention concerne un nanocorps dirigé contre VCAM-1 comprenant :
a) les séquences d'acides aminés (i) SEQ ID NO: 1 comme CDR1, (ii) SEQ ID NO: 2 comme CDR2 et (iii) SEQ ID NO: 3 comme CDR3; ou
b) les séquences d'acides aminés (i) SEQ ID NO: 4 comme CDR1, (ii) SEQ ID NO: 5 comme CDR2 et (iii) SEQ ID NO: 6 comme CDR3; ou
c) les séquences d'acides aminés (i) SEQ ID NO: 7 comme CDR1, (ii) SEQ ID NO: 8 comme CDR2 et (iii) SEQ ID NO: 9 comme CDR3; ou
d) les séquences d'acides aminés (i) SEQ ID NO: 10 comme CDR1, (ii) SEQ ID NO: 11 comme CDR2 et (iii) SEQ ID NO: 12 comme CDR3;
ou un variant fonctionnellement conservatif du nanocorps défini en a), b), c) ou d) comprenant une substitution conservative d'un ou deux acides aminés dans respectivement une, deux ou trois des séquences SEQ ID NO: 1, SEQ ID NO: 2 et SEQ ID NO: 3, ou SEQ ID NO: 4, SEQ ID NO: 5 et SEQ ID NO: 6, ou SEQ ID NO: 7, SEQ ID NO: 8 et SEQ ID NO: 9, ou SEQ ID NO: 10, SEQ ID NO: 11 et SEQ ID NO: 12.

Les inventeurs ont également séquencé les régions "charpente" (FR) de ces nanocorps. Les séquences correspondantes sont les suivantes :
- cAbVCAM1-5
   ∘ Région FR1 : QVQLQESGGGSVQTGGSLRLSCAASG (SEQ ID NO: 17)
   ∘ Région FR2 : WFRQAPGKKREGVA (SEQ ID NO: 18)
   ∘ Région FR3 : AYYAGSVKGRFTISHDNAKNTVYLQMNNLNPEDTAMYYCAA (SEQ ID NO: 19)
   ∘ Région FR4 : WGQGTQVTVSS (SEQ ID NO: 20)
- cAbVCAM1-3
   ∘ Région FR1 : QVQLQESGGGSVQAGGSLRLSCTASG (SEQ ID NO: 21)
   ∘ Région FR2 : WVRQAPGKGLEWVS (SEQ ID NO: 22)
   ∘ Région FR3 : TYYADSVKGRFTISRDNAKNTLYLQMNSLKSEDTALYYCAT (SEQ ID NO: 23)
   ∘ Région FR4 : KGQGTQVTVSS (SEQ ID NO: 24)
- cAbVCAM1-8
   ∘ Région FR1 : QVQLQESGGGLVQPGGSLRLSCAASG (SEQ ID NO: 25)
   ∘ Région FR2 : WVRRAPGKGLEWVS (SEQ ID NO: 26)
   ∘ Région FR3 : TLYLPSVKGRFTISRDNAKNTLYLQMNSLKSEDTGWYYCVE (SEQ ID NO: 27)
   ∘ Région FR4 : RGQGTQVTVSS (SEQ ID NO: 28)
- cAbVCAM1-9
   ∘ Région FR1 : QVQLQESGGGLVQPGGSLRLSCAASG (SEQ ID NO: 29)
   ∘ Région FR2 : WVRQAPGKGLEWVS (SEQ ID NO: 30)
   ∘ Région FR3 : TYYADSVKGRFTISRDNAKNTLYLQMNSLKSEDTALYYCAT (SEQ ID NO: 31)
   ∘ Région FR4 : KGQGTQVTVSS (SEQ ID NO: 32)

Dans un mode de réalisation particulier, l'invention concerne un nanocorps comprenant ou consistant en l'enchainement de séquences FR1-CDR1-FR2-CDR2-FR3-CDR3-FR4 telles que définies ci-dessus de l'un des nanocorps identifiés par les inventeurs.

De préférence, le nanocorps selon l'invention est donc un nanocorps comprenant ou consistant en une séquence d'acides aminés sélectionnée dans le groupe consistant en les séquences d'acides aminés SEQ ID NO: 13, SEQ ID NO: 14, SEQ ID NO: 15 et SEQ ID NO: 16, ou un variant fonctionnellement conservatif de celui-ci comprenant une substitution conservative d'un ou deux acides aminés dans un, deux ou trois des CDRs compris respectivement dans la séquence d'acides aminé SEQ ID NO: 13, SEQ ID NO: 14, SEQ ID NO: 15 ou SEQ ID NO: 16. Le variant fonctionnellement conservatif tel que défini ci-dessus peut en outre comprendre une ou plusieurs substitutions, en particulier une ou plusieurs substitutions conservatives dans les régions respectivement des séquences d'acides aminés SEQ ID NO: 16, SEQ ID NO: 14, SEQ ID NO: 15 ou SEQ ID NO: 16 qui ne sont pas des CDRs, telles que les régions "charpente", en particulier les régions "charpente" définies ci-dessus. De manière davantage préférée, le nanocorps selon l'invention est un nanocorps comprenant ou consistant en une séquence d'acides aminés sélectionnée dans le groupe consistant en les séquences d'acides aminés SEQ ID NO: 13, SEQ ID NO: 14, SEQ ID NO: 15 et SEQ ID NO: 16. De manière préférée entre toutes, le nanocorps selon l'invention est un nanocorps comprenant ou consistant en la séquence d'acides aminés SEQ ID NO: 13.

Dans le contexte de l'invention, l'expression "variant fonctionnellement conservatif" fait référence à des variants dans lesquels un acide aminé donné dans un nanocorps selon l'invention est substitué sans altérer la conformation globale et la fonction du nanocorps, incluant un remplacement d'un acide aminé par un autre ayant des propriétés similaires (par exemple polarité, potentiel de liaison hydrogène, acidité, basicité, hydrophobicité, présence d'un groupe aromatique, etc). Les acides aminés ayant des propriétés similaires sont bien connus de l'homme du métier. Par exemple, l'arginine, l'histidine et la lysine des acides aminés hydrophiles-basiques et peuvent être interchangeables. De manière similaire, l'isoleucine, un acide aminé hydrophobe, peut être remplacé par la leucine, la méthionine ou la valine. De tels changements devraient avoir peu ou pas d'effet sur le poids moléculaire apparent ou le point isoélectrique du nanocorps. Un acide aminé naturel peut être remplacé par un acide aminé non naturel, tel qu'un acide aminé en configuration D, acide aminé bêta ou gamma.

Des exemples de substitutions conservatives sont présentés dans le tableau 1 ci-dessous.

**Tableau 1: Substitutions conservatives I**

| **Caractéristique de la chaîne latérale** | **Acide aminé** |
|---|---|
| Non polaire | G A P I L V |
| Polaire non chargé | C S T M N Q |
| Polaire chargé | D E K R |
| Aromatique | H F W Y |
| Autre | N Q D E |

Alternativement, les acides amines conservatifs peuvent être groupés comme décrit dans Lehninger (1975, Biochemistry, 2nde Edition, Worth Publishers, Inc. New-York: NY., p. 71-77), comme montré dans le tableau 2 ci-dessous.

**Tableau 2: Substitutions conservatives II**

| **Caractéristique de la chaîne latérale** | | **Acide aminé** |
|---|---|---|
| Non polaire | Aliphatique | A L I V P |
| | Aromatique | F W |
| | Contenant du souffre | M |
| | Frontière | G |
| Polaire non chargé | Hydroxyle | S T Y |
| | Amides | N Q |
| | Sulfhydrile | C |
| | Frontière | G |
| Chargé positivement (basique) | | K R H |
| Chargé négativement (acide) | | D E |

Selon une autre alternative, des exemples de substitutions conservatives sont présentés dans le tableau 3 ci-dessous.

**Tableau 3: Substitutions conservatives III**

| **Résidu d'origine** | **Exemple de substitution** |
|---|---|
| A | V L I |
| R | K Q N |
| N | Q H K R |
| D | E |
| C | S |
| G | N |
| E | D |
| H | N Q K R |
| I | L V M A F |
| L | I V M A F |
| K | R Q N |
| M | L F I |
| F | L V I A |
| P | G |
| S | T |
| T | S |
| W | Y |
| Y | W F T S |
| V | I L M F A |

Ces variants fonctionnellement conservatifs conservent la capacité de lier VCAM-1. Préférentiellement ces variants fonctionnellement conservatifs présentent une affinité de liaison avec VCAM-1 égale ou accrue par rapport au nanocorps correspondant.

Connaissant la séquence d'acides aminés du nanocorps d'intérêt, l'homme du métier est capable de produire les nanocorps selon l'invention, en particulier les nanocorps cAbVCAM1-5, cAbVCAM1-3, cAbVCAM1-8 et cAbVCAM1-9 définis ci-dessus, par des techniques conventionnelles de production de polypeptides. Par exemple, ils peuvent être synthétisés en utilisant la méthode bien connue de synthèse en phase solide (Merrifield (19962) Proc. Soc. Ex. Boil. 21:412; Merrifield (1963) J. Am. Chem. Soc. 85:2149; Tam et al. (1983) J. Am. Chem. Soc. 105:6442), de préférence en utilisant un appareil de synthèse peptidique disponible commercialement (tel que celui fait par Applied Biosystems, Foster City, Californie) et en suivant les instructions du fabriquant.

Alternativement, les nanocorps selon l'invention peuvent être synthétisés par des techniques d'ADN recombinant bien connues de l'homme du métier (Maniatis et al. (1982) Molecular Cloning: a laboratory manual, Cold Spring Harbor Laboratories, NY, 51-54 et 412-430). Par exemple, ils peuvent être obtenus comme produits d'expression d'ADN après incorporation des séquences d'ADN codant le polypeptide d'intérêt dans des vecteurs d'expression et introduction de ces vecteurs dans les hôtes procaryotes ou eucaryotes appropriés qui exprimeront le polypeptide d'intérêt, à partir desquels ils pourront ensuite être isolés en utilisant des techniques bien connues de l'homme du métier. Comme il est bien connu de l'homme du métier, lorsqu'une protéine est synthétisée par des techniques d'ADN recombinant, elle est généralement synthétisée associée à une étiquette facilitant sa purification. De telles étiquettes sont bien connues de l'homme du métier et incluent par exemple l'hexahistidine (6His), la glutathion-S-transférase (GST) ou l'hémagglutinine du virus de la grippe (HA). De préférence, les nanocorps selon l'invention comprennent une étiquette hexahistidine. Par conséquent, un objet de l'invention est également constitué par un nanocorps comprenant ou étant constitué de la séquence d'acides aminés choisie dans le groupe constitué des séquences SEQ ID NO: 13, SEQ ID NO: 14, SEQ ID NO: 15 et SEQ ID NO: 16 comprenant en outre à leur extrémité C-terminale ou N-terminale, de préférence à leur extrémité C-terminale, six résidus histidine. Un nanocorps comprenant ou étant constitué de la séquence d'acides aminés choisie dans le groupe constitué des séquences SEQ ID NO: 33, SEQ ID NO: 34, SEQ ID NO: 35 et SEQ ID NO: 36 fait donc également partie de l'invention.

Un autre objet de l'invention concerne un acide nucléique comprenant une séquence nucléique codant le nanocorps selon la présente invention.

Dans un mode de réalisation particulier, l'acide nucléique selon l'invention comprend ou consiste en une séquence nucléique codant un nanocorps défini par l'une des séquences d'acides aminés SEQ ID NO: 13, SEQ ID NO: 14, SEQ ID NO: 15 ou SEQ ID NO: 16. De manière préférée, l'acide nucléique selon l'invention comprend ou consiste en une séquence nucléique codant le nanocorps défini par la séquence d'acides aminés SEQ ID NO: 13.

Typiquement, ledit acide nucléique est une molécule d'ADN ou d'ARN, qui peut être incluse dans n'importe quel vecteur approprié, tel qu'un plasmide, un cosmide, un épisome, un chromosome artificiel, un phage ou un vecteur viral.

Les termes "vecteur", "vecteur de clonage" et "vecteur d'expression" signifient le véhicule par lequel la séquence d'ADN ou d'ARN peut être introduite dans la cellule hôte, de manière à transformer l'hôte et promouvoir l'expression (e.g. transcription et traduction) de la séquence introduite.

Par conséquent, un autre objet de l'invention concerne un vecteur comprenant un acide nucléique selon l'invention.

De tels vecteurs peuvent comprendre des éléments régulateurs, tels qu'un promoteur, un activateur, un terminateur, etc., pour causer ou diriger l'expression du polypeptide. Des exemples de promoteurs et d'activateurs utilisés dans les vecteurs d'expression pour cellule animale incluent le promoteur précoce et l'activateur de SV40 (Mizukami et al. (1987) J. Biochem. 101:1307-1310), le promoteur LTR et l'activateur de virus de la leucémie de la souris Moloney, le promoteur (Mason et al (1985) Cell 14:479-487) et l'activateur (Gillies et al. (1983) Cell 33:717-728) de la chaîne de l'immunoglobuline, etc.

N'importe quel vecteur d'expression pour cellules animales peut être utilisé. Des exemples de vecteurs appropriés incluent pAGE107 (Miyaji et al. (1990) Cytotechnology 3:133-140), pAGE103 (Mizukami et al. (1987) J. Biochem. 101:1307-1310), pHSG274 (Brady et al. (1984) Gene 27:223-232), pKCR (O'Hare et al. (1981) Proc. Natl. Acad. Sci. USA 78:1527-1531), pSG1 beta d2-4 (Miyaji et al. (1990) Cytotechnology 3:133-140), etc.

D'autres exemples de plasmides incluent des plasmides réplicatifs comprenant une origine de réplication, ou des plasmides intégratifs, tels que par exemple pUC, pcDNA, pBR, etc.

D'autres exemples de vecteurs viraux incluent les vecteurs adénoviraux, rétroviraux, du virus de l'herpès et AAV. De tels virus recombinants peuvent être produits par des techniques bien connues de l'homme du métier, telles que par transfection des cellules d'empaquetage ou par transfection transitoire avec des plasmides ou virus auxiliaires. Des exemples typiques de cellules d'empaquetage de virus incluent les cellules PA317, les cellules PsiCRIP, les cellules GPenv+, les cellules 293, etc. Des protocoles détaillés de production de tels virus recombinants déficients pour leur réplication peuvent être trouvés par exemple dans les demandes WO 95/14785, WO 96/22378, US 5,882,887, US 6,013,516, US 4,861,719, US 5,278,056 et WO 94/19478.

Un autre objet de la présente invention concerne une cellule qui a été transfectée, infectée ou transformée avec un acide nucléique et/ou un vecteur selon l'invention.

Le termine "transformation" signifie l'introduction d'un gène ou d'une séquence d'ADN ou d'ARN "étranger" (*i.e.* extrinsèque ou extracellulaire) dans une cellule hôte, de telle sorte que la cellule hôte exprimera le gène ou la séquence introduit pour produire la substance d'intérêt, typiquement une protéine codée par le gène ou la séquence introduit. Une cellule hôte qui reçoit et exprime l'ADN ou l'ARN introduit a été "transformée".

Les acides nucléiques selon l'invention peuvent être utilisés pour produire un nanocorps selon l'invention dans un système d'expression approprié. Le terme "système d'expression" signifie une cellule hôte et un vecteur compatible dans des conditions appropriées, e.g. pour l'expression d'une protéine codée par l'ADN étranger porté par le vecteur et introduit dans la cellule hôte.

Des systèmes d'expression conventionnels incluent les cellules hôtes *Escherichia coli* et les vecteurs plasmidiques, les cellules hôtes d'insecte et les vecteurs Baculovirus, et les cellules hôtes de mammifère et leurs vecteurs. D'autres exemples de cellules hôtes incluent les cellules procaryotes (telles que les bactéries), et les cellules eucaryotes (telles que les cellules de levure, les cellules de mammifère, les cellules d'insecte, les cellules de plantes, etc.). Des exemples spécifiques incluent *Escherichia coli*, les levures *Kluyveromyces* ou *Saccharomyces,* les lignées cellulaires de mammifère (*e.g.* les cellules Vero, les cellules CHO, les cellules 3T3, les cellules COS, etc.) ainsi que les cultures de cellules de mammifères primaires ou établies (e.g. produites à partir de lymphoblastes, fibroblastes, cellules épithéliales, cellules nerveuses, adipocytes, etc.). Des exemples incluent également les cellules SP2/0-Ag14 de souris (ATCC CRL1581), les cellules P3X63-Ag8.653 de souris (ATCC CRL1580), les cellules CHO dans lesquelles un gène de la réductase dihydrofolate est défectueux, les cellules YB2/3HL.P2.G11.16Ag.20 de rat (ATCC CRL1662), etc.

La présente invention concerne également une méthode de production d'une cellule hôte recombinante exprimant un nanocorps selon l'invention, ladite méthode comprenant les étapes consistant à: (i) introduire *in vitro* ou *ex vivo* un acide nucléique ou un vecteur recombinant tel que décrit ci-dessus dans une cellule hôte compétente, (ii) cultiver *in vitro* ou *ex vivo* la cellule hôte recombinante obtenue et (iii) éventuellement sélectionner les cellules qui expriment et/ou sécrètent ledit nanocorps. De telles cellules hôtes recombinantes peuvent être utilisées pour la production de nanocorps selon l'invention.

Les nanocorps selon l'invention peuvent être produits par n'importe quelle technique connue de l'homme du métier, telles que par exemple n'importe quelle technique chimique, biologique, génétique ou enzymatique, seule ou en combinaison.

En particulier, l'invention concerne en outre une méthode de production d'un nanocorps selon l'invention, ladite méthode comprenant les étapes consistant à : (i) cultiver une cellule hôte transformée selon l'invention dans des conditions appropriées pour permettre l'expression dudit nanocorps, et (ii) récupérer le nanocorps exprimé.

Les nanocorps selon l'invention peuvent être convenablement séparés du milieu de culture par des procédures de purification d'immunoglobulines conventionnelles, telles que par exemple protéine A-Sépharose, chromatographie hydroxilapatite, électrophorèse sur gel, dialyse ou chromatographie d'affinité.

### Nanocorps marqués

Les nanocorps selon l'invention sont particulièrement utiles pour l'imagerie médicale. Dans ce contexte, il est particulièrement intéressant de disposer de nanocorps associés à un marqueur détectable. Les présents inventeurs ont montré que les nanocorps cAbVCAM1-5, cAbVCAM1-3, cAbVCAM1-8 et cAbVCAM1-9 conservaient leurs propriétés lorsqu'ils étaient associés à un marqueur détectable, en particulier un radioélément tel que le technétium 99m.

La présente invention concerne donc également un nanocorps tel que défini ci-dessus associé à un marqueur détectable.

Par "nanocorps associé à un marqueur détectable", on entend ici que le marqueur détectable est lié, de manière directe ou indirecte, au nanocorps, ou est incorporé dans le nanocorps. Le marqueur détectable peut en particulier être lié au nanocorps par substitution (par exemple, en substituant un H par un I au niveau des résidus tyrosine), par complexion ou par chélation.

Par "marqueur détectable", on entend ici un composé qui produit un signal détectable. Lorsqu'il est associé à un traceur, il permet de suivre le devenir du traceur dans l'organisme. Le marqueur détectable peut être un agent de contraste IRM, un agent de contraste en scintigraphie, un agent de contraste en imagerie aux rayons X, un agent de contraste ultrason, un agent de contraste en imagerie optique. Des exemples de marqueurs détectables incluent des radioéléments, des fluorophores tels que la fluorescéine, l'Alexa, la cyanine; les composés chimioluminescents tels que le luminol; les composés bioluminescents tels que la luciférase ou la phosphatase alcaline; et les agents de contraste tels que les nanoparticules ou le Gadolinium. Le choix du marqueur détectable approprié, qui dépend du système de détection utilisé, est à la portée de l'homme du métier. A titre d'exemple lorsque le système de détection est l'IRM, le marqueur détectable est de préférence une nanoparticule d'oxyde de fer ou du Gadolinium; lorsque le système de détection est l'imagerie par fluorescence, le marqueur détectable est de préférence la Fluorescéine, l'Alexa ou la cyanine; lorsque le système de détection est l'imagerie par chimioluminescence, le marqueur détectable est de préférence le luminol; lorsque le système de détection est l'imagerie par bioluminescence, le marqueur détectable est de préférence la luciférase ou la phosphatase alcaline; lorsque le système de détection est l'imagerie nucléaire, le marqueur détectable est de préférence un radioélément tel que le Fluor 18 (¹⁸F) pour l'imagerie TEP, ou le technetium 99m (^{99m}Tc) pour l'imagerie SPECT.

De préférence, le marqueur détectable est un radioélément. Des exemples de radioéléments, qui sont plus particulièrement utilisés dans les techniques d'imagerie nucléaires, incluent le Technetium 99m (^{99m}Tc), l'iode 123 (¹²³I), l'iode 125 (¹²⁵I), l'indium 111 (¹¹¹In), le fluor 18 (¹⁸F), le gallium 67 (⁶⁷Ga), le gallium 68 (⁶⁸Ga), le cuivre 64 (⁶⁴Cu) et n'importe quel autre radioélément utilisable chez les êtres humains. Par conséquent, de préférence, le radioélément est choisi dans le groupe constitué du ^{99m}Tc, ¹²³I, ¹²⁵I, ¹¹¹In, ¹⁸F, ⁶⁴Cu, ⁶⁷Ga et ⁶⁸Ga. De manière préférée entre toutes, le radioélément est le ^{99m}Tc.

### Utilisation comme agent de contraste

Les inventeurs ont démontré que les nanocorps cAbVCAM1-5, cAbVCAM1-3, cAbVCAM1-8 et cAbVCAM1-9 constituaient des traceurs spécifiques de la plaque d'athérome, en particulier de la plaque aortique, et permettent sa détection par imagerie.

L'invention propose donc un nanocorps tel que défini ci-dessus pour son utilisation comme agent de contraste dans l'imagerie médicale, en particulier l'imagerie médicale *in vivo,* non invasive.

Elle concerne également l'utilisation d'un nanocorps tel que défini ci-dessus pour la fabrication d'un agent de contraste utile pour l'imagerie médicale, en particulier l'imagerie médicale *in vivo,* non invasive.

Par "agent de contraste", on entend ici une substance ou une composition qui, administrée dans l'organisme, permet de marquer de manière détectable des organes ou des structures (tissu, cellule, récepteur) qui, sans agent de contraste, sont peu ou non visibles en imagerie médicale. Par extension, l'expression "agent de contraste" est utilisée pour désigner un traceur associé à un marqueur tel que défini ci-dessus.

Dans le contexte de l'invention, les "méthodes d'imagerie" se réfèrent à des méthodes qui permettent de visualiser l'intérieur d'un organisme ou des organes d'un organisme. Des exemples de méthodes d'imagerie incluent des techniques invasives telles que l'échographie endocoronaire, et des techniques non-invasives telles que l'imagerie par résonnance magnétique, l'imagerie aux rayons X, l'échographie, l'imagerie optique, ou la médecine nucléaire telle que la scintigraphie, en particulier la tomographie d'émission monophotonique (SPECT) et la tomographie d'émission de positons (PET). De préférence, la méthode d'imagerie selon l'invention est la scintigraphie, en particulier la scintigraphie SPECT ou PET.

La scintigraphie repose sur l'administration (généralement par voie intraveineuse) d'un agent de contraste, aussi appelé radio-pharmaceutique, constitué d'un traceur marqué par un radioélément. La localisation spécifique de cet agent de contraste dans l'organisme est ensuite déterminée par détection des rayons gamma ou bêta émis.

La tomographie d'émission monophotonique et la tomographie d'émission de positons sont des techniques d'imagerie médicale nucléaire tomographique basées sur la scintigraphie et qui permettent de réaliser des images et des reconstructions en trois dimensions des organes et de leur métabolisme au moyen d'un ensemble de caméras qui tournent autour du patient.

La présente invention concerne également une méthode d'imagerie médicale, en particulier d'imagerie médicale *in vivo,* non invasive, dans laquelle un nanocorps tel que défini ci-dessus est administré à un patient. La méthode d'imagerie médicale selon l'invention peut en outre comprendre les étapes de détection de la liaison ou de l'absence de liaison du nanocorps dans des zones corporelles du patient et de visualisation des zones corporelles du patient dans lesquelles la liaison du nanocorps peut être détectée.

Dans le contexte de l'invention, un "patient" désigne un mammifère humain ou non-humain, tel qu'un rongeur (rat, souris, lapin), un primate (chimpanzé), un félin (chat), un canin (chien). De préférence, l'individu est humain.

N'importe quelle méthode d'administration, connue de l'homme du métier, peut être utilisée pour administrer le nanocorps selon l'invention au patient. En particulier, le nanocorps peut être administré par exemple par voie orale, par inhalation or par voie parentérale (en particulier par injection intraveineuse). Quand la voie parentérale est choisie, le nanocorps peut être sous forme de solutions et de suspensions injectables, conditionné en ampoules ou flacons. Les formes d'administration parentérale sont conventionnellement obtenues par mélange du nanocorps selon l'invention avec des tampons, stabilisants, conservateurs, agents solubilisants, agents isotoniques et agents de mise en suspension. Selon des techniques connues, ces mélanges peuvent être stérilisés et conditionnés sous la forme d'injections intraveineuses. L'homme du métier peut par exemple utiliser des tampons à base de sels de phosphate en tant que tampons. Des exemples d'agents de mise en suspension incluent la méthylcellulose, l'acacia, et la carbocyméthylcellulose de sodium. Des exemples de stabilisants incluent le sulfite de sodium et le métasulfite de sodium, et des exemples de conservateurs incluent le p-hydroxybenzoate de sodium, l'acide sorbique, le crésol et le chlorocrésol.

La quantité de nanocorps administrée dépend naturellement de la voie d'administration, de la taille et/ou du poids du patient, et de la technique de détection utilisée.

Dans le contexte de l'invention, le terme "zone corporelle" fait référence à une région déterminée de l'organisme. Il peut s'agir par exemple d'un organe, d'une partie d'un organe ou d'un tissu, tel qu'un poumon, le coeur, le foie, la rate ou un rein, ou un vaisseau sanguin tel qu'une artère ou une veine, en particulier l'aorte ou les artères coronaires.

Dans un mode de réalisation particulier, le nanocorps selon l'invention est utilisé comme agent de contraste dans l'imagerie médicale pour visualiser des plaques athérosclérotiques, plus particulièrement des plaques athérosclérotiques aortiques, carotidiennes ou coronaires, dans un patient.

Dans le contexte de l'invention, les termes "plaque athérosclérotique", "plaque d'athérosclérose" et "plaque d'athérome" sont utilisés indifféremment et font référence à une lésion des parois des vaisseaux. De préférence, une plaque athérosclérotique comprend un coeur lipidique et une chape fibreuse, la chape étant constituée de cellules de muscle lisse, de collagènes et d'une matrice extracellulaire et isolant la coeur lipidique de la lumière artérielle. Les plaques athérosclérotiques peuvent se trouver par exemple dans l'aorte, la carotide ou dans l'artère coronaire. Quand la plaque comprend une chape fibreuse fine (environ 65 à 150 µm d'épaisseur) et un coeur lipidique important, elle est appelée "plaque vulnérable" ou "plaque instable". Ces plaques instables, qui présentent une tendance à la rupture, se rencontrent au niveau des artères coronaires et au niveau de l'aorte et de ses branches. La rupture des plaques coronariennes vulnérables provoque des "syndromes coronariens aigus". En cas de thrombose occlusive complète, il s'agit de l'infarctus du myocarde. Lorsque la thrombose de l'artère reste incomplète, il s'agit de l'angor instable. Au niveau de la carotide, les plaques vulnérables sont plus sténotiques et moins inflammatoires. Elles expriment également VCAM-1.

De préférence, le nanocorps selon l'invention est utilisé comme agent de contraste dans l'imagerie médicale pour visualiser des plaques athérosclérotiques vulnérables, en particulier des plaques vulnérables aortiques ou coronariennes.

### Utilisation diagnostique

L'apparition de plaques d'athérome est une marque de l'athérosclérose, qui constitue en elle-même une maladie cardiovasculaire et peut générer différentes complications cardiovasculaires. La détection de plaques d'athérome constitue donc la caractérisation d'une maladie cardiovasculaire. D'autre part, la possibilité de suivre par imagerie l'évolution, c'est-à-dire la progression ou la régression, d'une plaque d'athérome précédemment identifiée représente une modalité pour évaluer l'efficacité d'un traitement thérapeutique chez un patient chez qui on a diagnostiqué une maladie cardiovasculaire.

L'invention concerne donc également un nanocorps tel que défini ci-dessus pour son utilisation dans des méthodes de diagnostic ou de pronostic.

Par "méthode diagnostique" ou "diagnostic", on entend ici une méthode permettant de déterminer si un individu souffre d'une pathologie.

Par "méthode pronostique" ou "pronostic", on entend ici une méthode permettant de déterminer si un individu risque de développer une pathologie.

De préférence, le nanocorps tel que défini ci-dessus est utilisé pour le diagnostic de maladies cardiovasculaires.

Dans le contexte de la présente invention, on désigne par "maladie cardiovasculaire" une maladie, une lésion, ou un symptôme lié à un processus d'athérogenèse affectant le système cardiovasculaire. Cela inclut notamment les états marquant le développement d'une plaque d'athérome (les plaques sont classifiées en stades de progression I à VI, selon la classification internationale de Stary), ainsi que les complications découlant de la formation d'une plaque d'athérome (sténose, ischémie) et/ou de son évolution vers un accident ischémique aigu (thrombose, embolie, infarctus, rupture artérielle). Les maladies cardiovasculaires désignent par exemple l'athérosclérose, une plaque d'athérome, en particulier la plaque vulnérable, la maladie coronarienne, l'angor, la thrombose, l'accident vasculaire cérébral, l'infarctus du myocarde, la sténose vasculaire, l'infarctus. De préférence, le nanocorps selon l'invention est utilisé pour diagnostiquer l'athérosclérose ou une maladie coronarienne.

Par "athérosclérose", on entend ici une maladie affectant les vaisseaux sanguins artériels. L'athérosclérose peut être caractérisée par une réponse inflammatoire chronique au niveau des parois des artères, principalement due à l'accumulation de macrophages et promue par des lipoprotéines de faible densité.

La "maladie coronarienne" est la manifestation la plus courante de la maladie cardiovasculaire. Il s'agit d'une maladie progressive, due à une mauvaise irrigation du muscle cardiaque, consécutive au rétrécissement (sténose) ou à la calcification (sclérose) d'une ou des artères coronaires. Le symptôme principal de la maladie coronarienne se manifeste sous la forme de douleurs qui constituent un angor (stable ou instable), aussi appelé angine de poitrine. L'obstruction complète d'une ou des artères coronaires conduit à l'infarctus.

L"'infarctus" désigne un foyer circonscrit de nécrose dû à une obstruction artérielle. Plus spécifiquement, l'infarctus du myocarde est une nécrose du myocarde qui résulte généralement d'une thrombose coronaire aiguë, secondaire à une rupture de plaque (généralement une plaque vulnérable) entraînant l'agrégation plaquettaire puis l'occlusion coronaire.

La présence d'une plaque d'athérome coronaire, surtout s'il s'agit d'une plaque instable, expose le sujet à un risque d'accident ischémique aigu, notamment un infarctus du myocarde. Le nanocorps selon l'invention peut donc être utilisé pour détecter un risque de survenue d'un accident ischémique aigu, en particulier un risque de survenue d'un infarctus du myocarde, chez un patient.

Par "risque de survenue", on entend ici la probabilité qu'un individu développe une pathologie.

L"'accident ischémie aigu" désigne la diminution de l'apport sanguin artériel dans un territoire de l'organisme. Ses principales causes locales sont la thrombose et l'embolie.

La "thrombose" correspond à la coagulation du sang dans les cavités vasculaires (artères, veines, capillaires ou cavités cardiaques) conduisant à la formation d'un thrombus.

L"'embolie" est la migration d'un corps étranger, le plus souvent constitué par un caillot sanguin (thrombus), et son arrêt brusque dans un vaisseau dont le calibre est insuffisant pour le laisser passer. Les conséquences locales de l'embolie sont des perturbations circulatoires liées à l'obstruction vasculaire, conduisant le plus souvent à un infarctus.

La plaque d'athérome peut aussi être localisée au niveau d'une artère carotide. Ces lésions conduisent à des accidents vasculaires cérébraux, hémorragiques (rupture d'anévrisme) ou ischémiques (infarctus cérébral). Le nanocorps selon l'invention peut donc être utilisé pour détecter un risque de survenue d'un accident vasculaire cérébral chez un patient.

Il peut encore s'agir d'une plaque d'athérome localisée au niveau d'une artère rénale, le rein étant l'un des organes cibles de l'athérosclérose. Une sténose important peut conduire à une hypertension artérielle et/ou une insuffisance rénale. L'atteinte athéromateuse des artères rénales peut également conduire à un accident vasculaire aigu, l'embolie rénale. Le nanocorps selon l'invention peut donc également être utilisé pour détecter un risque de survenue d'une embolie rénale chez un patient.

Les plaques d'athérome peuvent être localisées au niveau des artères des membres inférieurs (risque d'ischémie aiguë d'un membre), ou de l'aorte (risque de rupture d'anévrisme/dissection aortique). Le nanocorps selon l'invention peut donc être utilisé pour détecter un risque de survenue d'une ischémie d'un membre ou de rupture d'anévrisme aortique chez un patient.

Par conséquent, le nanocorps tel que défini ci-dessus est préférablement pour un utilisation pour détecter le risque de survenue d'un accident ischémique aigu sélectionné dans le groupe constitué de l'infarctus du myocarde, l'accident vasculaire cérébral, l'embolie artérielle rénale et l'accident ischémique aigu d'un membre.

La présente invention concerne également une méthode, en particulier une méthode *in vitro,* de diagnostic d'une maladie cardiovasculaire et/ou de détection d'un risque de survenue d'un accident ischémique aigu chez un patient, ladite méthode comprenant les étapes consistant à administrer un nanocorps tel que défini ci-dessus audit patient et à détecter ledit nanocorps dans l'organisme dudit patient, la détection d'une localisation préférentielle dudit nanocorps au niveau du système cardiovasculaire étant indicatrice d'une maladie cardiovasculaire et/ou d'un risque de survenue d'un accident ischémique aigu.

Par "localisation préférentielle", on entend que la quantité de nanocorps détectée au niveau du système cardiovasculaire est supérieure au bruit de fond qui correspond à une localisation non spécifique du nanocorps dans l'organisme.

L'invention concerne également le nanocorps tel que défini ci-dessus pour son utilisation pour le suivi thérapeutique d'une maladie cardiovasculaire chez un sujet chez qui une maladie cardiovasculaire a été diagnostiquée.

Elle concerne aussi l'utilisation du nanocorps tel que défini ci-dessus pour la fabrication d'un agent de contraste utile pour le suivi thérapeutique d'une maladie cardiovasculaire chez un sujet chez qui une maladie cardiovasculaire a été diagnostiquée.

Par "suivi thérapeutique" on entend ici l'observation de la réponse du sujet au traitement qui lui est administré. L'effet thérapeutique d'un traitement est généralement associé à un ralentissement ou une inhibition de la progression d'une maladie, à une réversion de la maladie, ou d'un ou plusieurs symptômes associés à cette maladie. Inversement, une absence d'effet thérapeutique peut se traduire par une stabilité, voire une accélération, de la progression de la maladie ou d'un ou plusieurs de ses symptômes.

Par exemple si la maladie cardiovasculaire au sens de l'invention est une plaque d'athérome, le suivi thérapeutique peut être réalisé en observant la disparition, la régression, le maintien, ou la croissance de la plaque d'athérome. Ainsi l'utilisation selon l'invention peut comprendre les étapes consistant à :
a) administrer un nanocorps tel que défini ci-dessus à un sujet chez qui on a détecté une plaque d'athérome ;
b) détecter la liaison du nanocorps au niveau de ladite plaque d'athérome ;
c) répéter les étapes a) et b) avant et après administration d'un traitement audit sujet ;
une absence ou une diminution de la liaison du nanocorps au niveau de ladite plaque étant indicatrice d'un traitement ayant un effet thérapeutique.

### Utilisation comme médicament

L'invention concerne en outre l'utilisation d'un nanocorps tel que défini ci-dessus pour la fabrication d'un médicament, en particulier un médicament destiné au traitement d'une maladie cardiovasculaire. Une méthode de traitement comprenant l'administration d'une quantité thérapeutiquement efficace du nanocorps tel que défini ci-dessus à un patient en ayant besoin, fait également partie de la présente invention.

L'utilisation d'un ligand de VCAM-1, seul ou couplé à un agent stabilisateur des plaques d'athérome, permet en effet de réduire la probabilité de rupture des plaques.

Par "traitement" d'une maladie cardiovasculaire on entend le "traitement thérapeutique" (ou curatif) d'une maladie cardiovasculaire, qui inclut le ralentissement ou l'inhibition de l'évolution d'une plaque d'athérosclérose, en particulier vers un stade de plaque d'athérosclérose vulnérable, ou la régression d'une plaque d'athérosclérose, en particulier d'une plaque vulnérable. On entend également le "traitement prophylactique" d'une maladie cardiovasculaire qui inclut notamment la prévention d'un accident ischémique aigu.

Le nanocorps selon l'invention est avantageusement couplé à un agent stabilisateur des plaques d'athérome, de manière à mettre en contact l'agent stabilisateur avec la lésion. Le choix d'un agent stabilisateur approprié est à la portée de l'homme du métier. Des exemples d'agents stabilisateurs incluent notamment des molécules anti-inflammatoires, telles que les anti-inflammatoires non stéroïdiens.

L'invention concerne plus particulièrement le nanocorps tel que défini ci-dessus pour son utilisation pour le traitement de l'athérosclérose et/ou à la prévention d'un accident ischémique aigu, de préférence pour le traitement d'une plaque athérosclérotique vulnérable.

L'invention concerne également l'utilisation d'un nanocorps tel que défini ci-dessus pour la fabrication d'un médicament destiné au traitement de l'athérosclérose et/ou à la prévention d'un accident ischémique aigu chez un patient susceptible de présenter une maladie cardiovasculaire.

L'invention concerne également une méthode de traitement de l'athérosclérose et/ou de prévention d'un accident ischémique aigu chez un patient en ayant besoin, comprenant l'administration d'une quantité thérapeutiquement efficace d'un nanocorps tel que défini ci-dessus à un patient en ayant besoin.

Préférentiellement, le nanocorps selon l'invention est utilisé pour traiter une plaque d'athérome vulnérable, de préférence encore une plaque d'athérome coronaire ou aortique vulnérable.

Préférentiellement, ledit accident ischémique aigu est sélectionné dans le groupe constitué d'un infarctus du myocarde, un accident vasculaire cérébral, une embolie rénale, une ischémie aiguë d'un membre, et une rupture d'anévrysme aortique.

Le nanocorps selon l'invention peut être administré par exemple par voie orale, par inhalation, par voie parentérale (en particulier par injection intraveineuse), sous une forme appropriée. Lorsque la voie parentérale est envisagée, le nanocorps peut être sous la forme de solutés et suspensions injectables conditionnées en ampoules ou flacons. Les formes pour l'administration parentérale sont obtenues de façon conventionnelle par mélange du nanocorps avec des tampons, des agents stabilisants, des conservateurs, des agents solubilisants, des agents isotoniques et des agents de mise en suspension. Conformément aux techniques connues, ces mélanges sont ensuite stérilisés puis conditionnés sous la forme d'injections intraveineuses. A titre de tampon, l'homme du métier pourra utiliser des tampons à base de sels de phosphate organique. Des exemples d'agents de mise en suspension englobent la méthylcellulose, l'hydroxyéthylcellulose, l'hydroxy-propylcellulose, l'acacia et la carboxyméthylcellulose sodique. En outre, des stabilisants utiles selon l'invention sont le sulfite de sodium et le métasulfite de sodium, tandis que l'on peut citer le p-hydroxybenzoate de sodium, l'acide sorbique, le crésol et le chlorocrésol en tant que conservateurs.

La quantité de nanocorps administrée dépend naturellement du mode d'administration, de la taille et/ou du poids du patient, et de la nature de l'agent cytotoxique qui peut lui être associé.

La présente invention concerne également une composition pharmaceutique comprenant un nanocorps tel que défini ci-dessus en association avec un véhicule pharmaceutiquement acceptable.

Le terme "pharmaceutiquement" ou "pharmaceutiquement acceptable" se réfère à des entités moléculaires et compositions qui ne produisent pas de réactions secondaires, allergiques ou autrement fâcheuses lorsqu'elles sont administrées à un mammifère, en particulier un humain.

Dans le contexte de l'invention, l'expression "véhicule pharmaceutiquement acceptable" inclut n'importe quel solvant, milieu de dispersion, revêtement, agent antibactérien ou antifongique, agent isotonique ou retardant l'absorption, et analogues. L'utilisation de tels milieux et agents pour les substances pharmaceutiquement actives est bien connue de l'homme du métier. A l'exception du cas où un milieu ou agent conventionnel est incompatible avec l'ingrédient actif, son utilisation dans les compositions pharmaceutiques est envisagée. Des ingrédients actifs supplémentaires peuvent également être incorporés dans les compositions.

### Utilisation pour détecter in vitro VCAM-1

La présente invention concerne également l'utilisation d'un nanocorps tel que défini ci-dessus pour la détection *in vitro* de VCAM-1 dans un échantillon.

Par "échantillon", on entend ici une partie d'un élément plus grand. De préférence, l'échantillon est une substance d'origine biologique. Des exemples d'échantillons biologiques incluent, mais ne sont pas limités à, des parties d'organes ou de tissus tels que le rein, le foie, le coeur, le poumon, etc., les artères, les veines, etc., le sang et ses composés tels que le plasma, les plaquettes, les sous-populations de cellules sanguines, etc.

La présente invention sera illustrée plus en détail par les figures et exemples ci-dessous.

### Description des figures

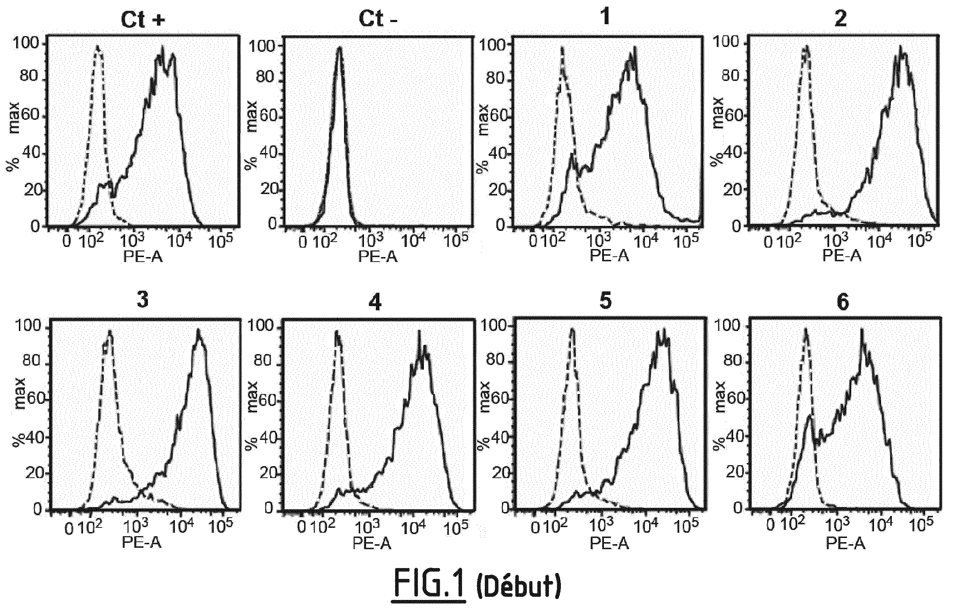

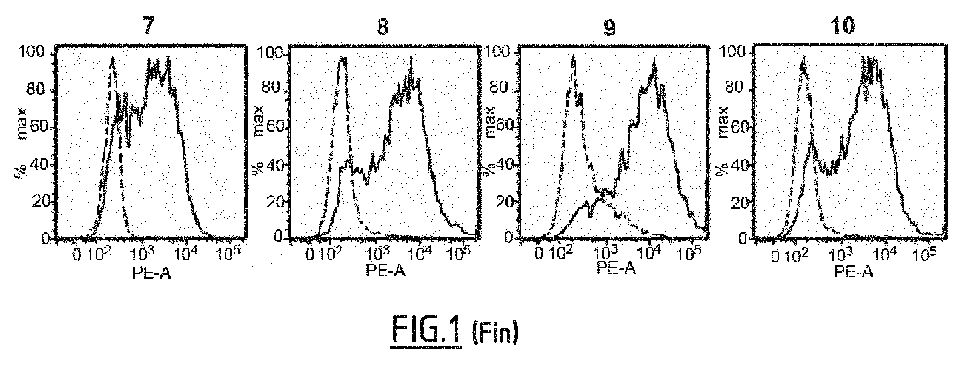

La **Figure 1** présente l'analyse en cytométrie en flux des nanocorps anti-VCAM-1 (cAbVCAM1-1 : 1; cAbVCAM1-2 : 2; cAbVCAM1-3 : 3; cAbVCAM1-4 : 4; cAbVCAM1-5 : 5; cAbVCAM1-6 : 6; cAbVCAM1-7 : 7; cAbVCAM1-8 : 8; cAbVCAM1-9 : 9 et cAbVCAM1-10 : 10) sur des cellules de souris bEND5 non traitées (trait pointillé) ou traitées au TNFα (trait plein). Un anticorps monoclonal anti-VCAM-1 de souris marqué au PE (Ct +) a été utilisé comme contrôle positif et le nanocorps cAbBcII10 (Ct -) a été utilisé comme contrôle négatif.

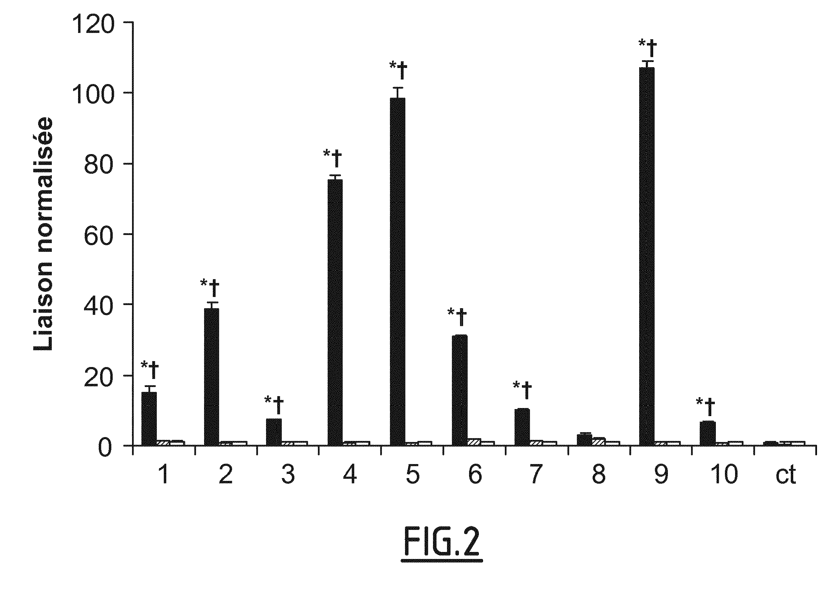

La **Figure 2** représente des histogrammes montrant la liaison normalisée des nanocorps anti-VCAM-1 (cAbVCAM1-1 : 1; cAbVCAM1-2 : 2; cAbVCAM1-3 : 3; cAbVCAM1-4 : 4; cAbVCAM1-5 : 5; cAbVCAM1-6 : 6; cAbVCAM1-7 : 7; cAbVCAM1-8 : 8; cAbVCAM1-9 : 9 et cAbVCAM1-10 : 10) marqués au ^{99m}Tc à des cellules bEND5 non traitées (barres blanches), traitées au TNFα (et exprimant donc VCAM-1; barres noires) ou traitées au TNFα et incubées avec un excès de nanocorps anti-VCAM-1 non marqué (barres grises). * : P<0,05 *versus* ^{99m}Tc-AbBcII10. † : P<0,05 *versus* traitées au TNFα et incubées avec un excès de nanocorps anti-VCAM-1 non marqué.

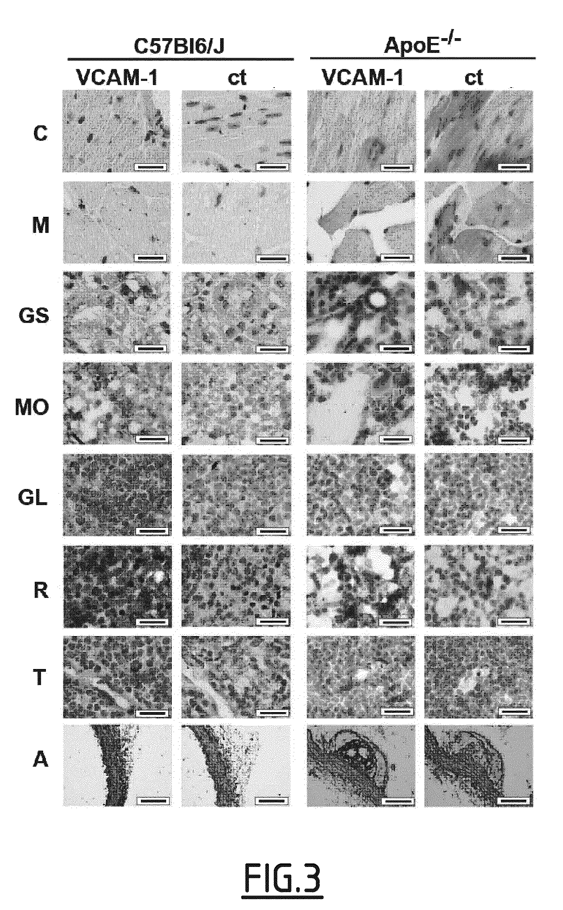

La **Figure 3** montre les résultats d'immunohistochimie sur des échantillons de coeur (C), muscle (M), glande salivaire (GS), de tissus lymphatiques incluant des échantillons de moelle osseuse (MO), ganglion lymphatique (GL), rate (R) et thymus (T), et des échantillons d'aorte (A) de souris contrôle C57BI/6J et de souris ApoE^{-/-} marquées avec un nanocorps anti-VCAM-1 (VCAM-1). La spécificité des résultats est montrée par l'absence de marquage en l'absence d'anticorps primaire (ct). La barre d'échelle représente 20 µm, sauf pour l'aorte (A) où elle représente 100 µm.

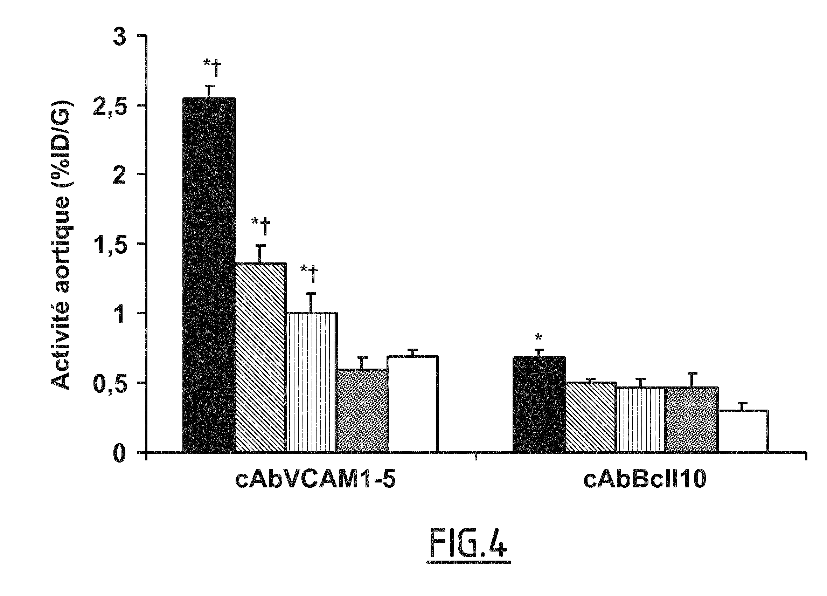

La **Figure 4** représente des histogrammes montrant l'absorption aortique (en %ID/g) du nanocorps cAbVCAM1-5 et du nanocorps contrôle cAbBcII10 dans l'aorte de souris contrôles C57BI/6J (barres blanches) et les segments artériels de souris ApoE^{-/-} rangées selon l'index d'extension de la lésion (+++: barres noires; ++ : barres à hachures diagonales; + : barres à hachures verticales; - : barres pointillées). Les résultats sont exprimés en moyenne ± erreur standard à la moyenne. * : P<0,05 *versus* C57BI/6J. † : P<0,05 *versus* index d'extension de la lesion suivant.

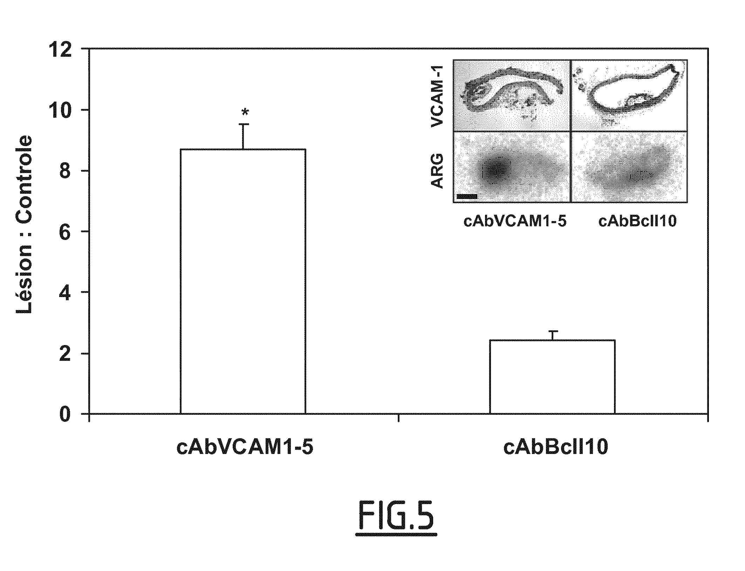

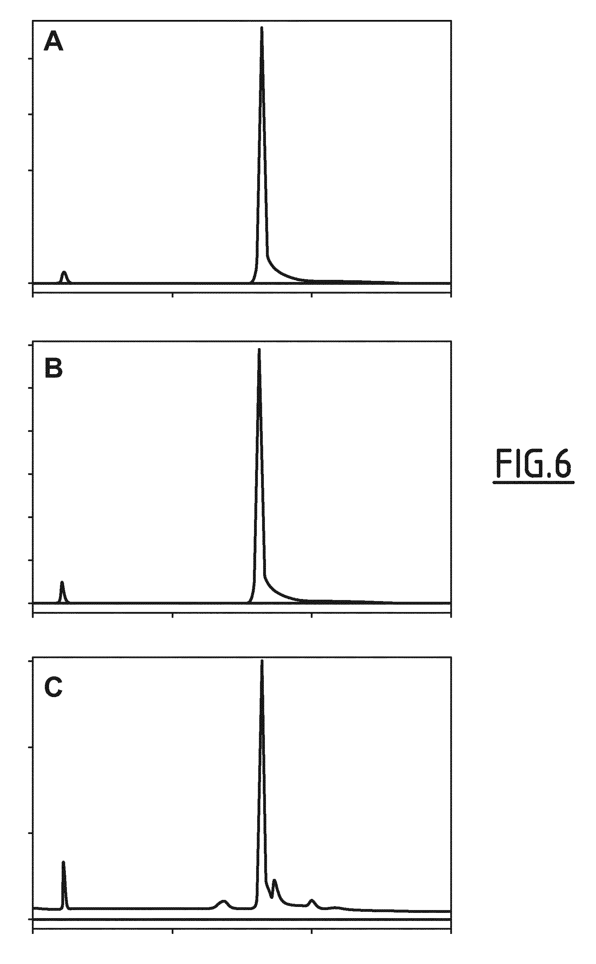

La **Figure 5** représente des histogrammes montrant le rapport lésion sur contrôle obtenu avec le nanocorps cAbVCAM1-5 et le nanocorps contrôle cAbBcII10 par quantification des autoradiogrammes. * : P<0,05 *versus* cAbBcII10. Des autoradiogrammes représentatifs (ARG) obtenus avec ^{99m}Tc-cAbVCAM1-5 et ^{99m}Tc-cAbBcII10 sur des lames adjacentes, dans lesquelles VCAM-1 a en outre été immunomarqué (VCAM-1) sont également présentés en haut à droite de la figure. La barre d'échelle représente 200 µm.
La **Figure 6** représente des profils HPLC du nanocorps ^{99m}Tc-cAbVCAM1-5 montrant que le nanocorps est stable *in vitro* 0 heure (A) et 6 heures (B) après le radiomarquage, ainsi *qu'in vivo* dans le sang 3 heures après injection (C).

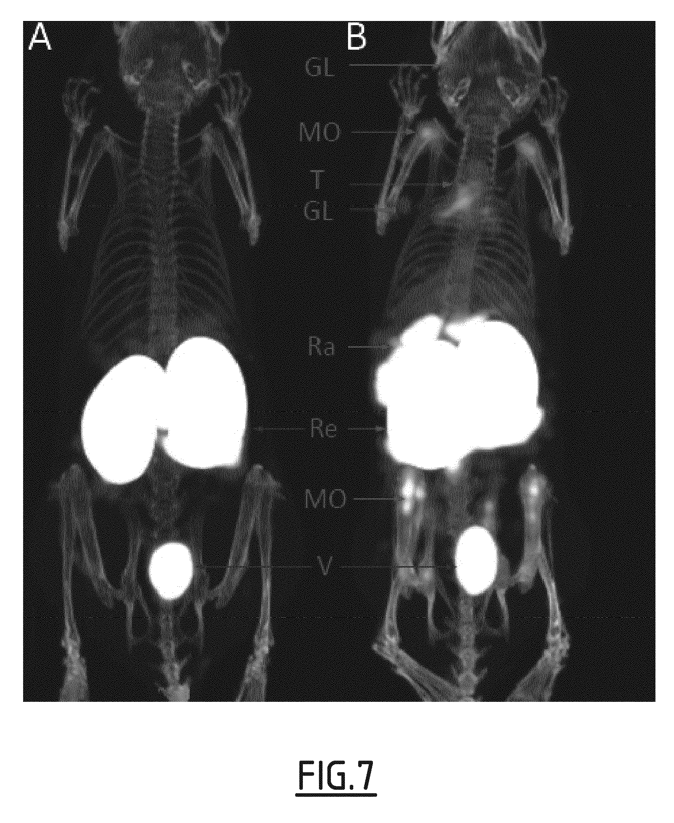

La **Figure 7** représente les images de projections d'intensité maximum (MIP) des nanocorps contrôle ^{99m}Tc-cAbBcII10 (A) et ^{99m}Tc-cAbVCAM1-5 (B) obtenues sur corps entier par SPECT/CT *in vivo* 3 heures après injection intraveineuse dans des souris C57BI/6J. La vessie (V), les reins (Re), les ganglions lymphatiques (GL), la moelle osseuse (MO), le thymus (T) et la rate (Ra) sont indiqués.

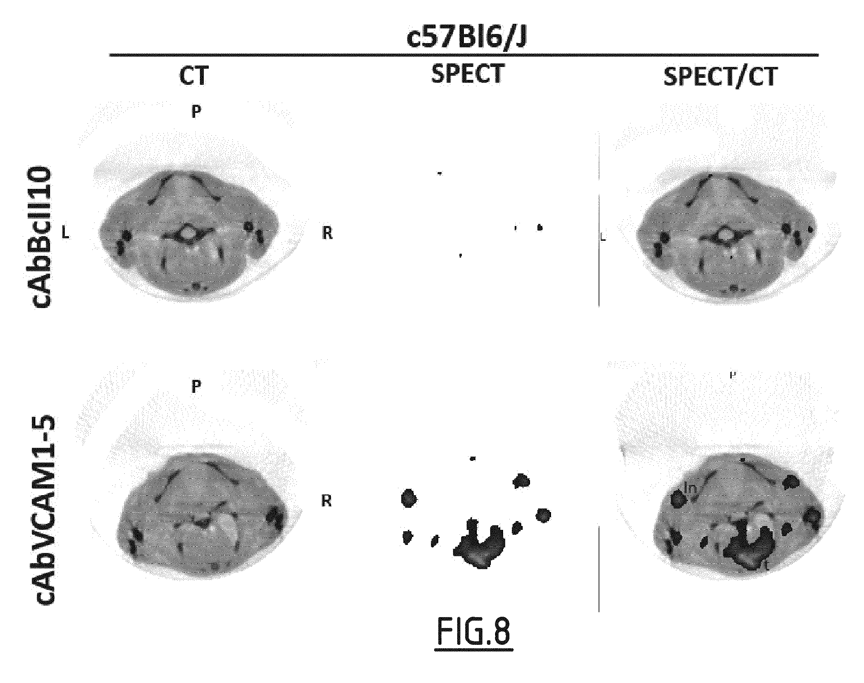

La **Figure 8** représente des vues coronaires représentatives en imagerie CT, SPECT et fusion SPECT/CT *in vivo,* prises au niveau de la crosse aortique de souris C57BI/6J, 3 heures après injection de nanocorps ^{99m}Tc-cAbBcII10 (cAbBcII10) ou de ^{99m}Tc-cAbVCAM1-5 (cAbVCAM1-5). L'échelle des images SPECT a été ajustée de 1 à 3,4% de la dose injectée de manière à permettre une comparaison directe. Les ganglions lymphatiques axillaires (In) et le thymus (t) sont indiqués sur les images SPECT/CT. L représente la gauche, R représente la droite, P représente la face postérieure.

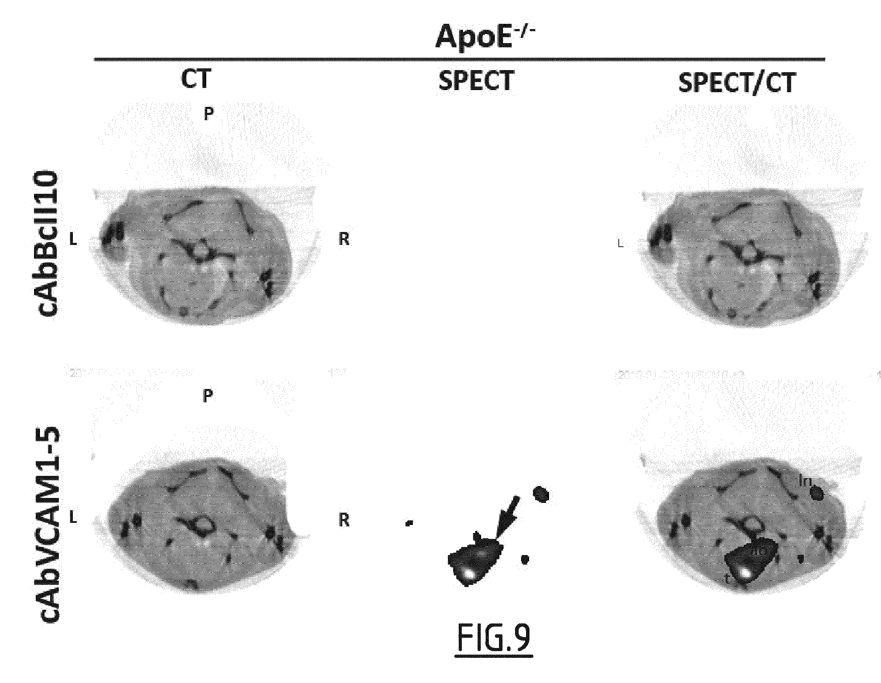

La **Figure 9** représente des vues coronaires représentatives en imagerie CT, SPECT et fusion SPECT/CT *in vivo,* prises au niveau de la crosse aortique de souris ApoE^{-/-}, 3 heures après injection de nanocorps ^{99m}Tc-cAbBcII10 (cAbBcII10) ou de ^{99m}Tc-cAbVCAM1-5 (cAbVCAM1-5). L'échelle des images SPECT a été ajustée de 1 à 3,4% de la dose injectée de manière à permettre une comparaison directe. Les ganglions lymphatiques axillaires (In), le thymus (t) et la crosse aortique (ao) sont indiqués sur les images SPECT/CT. La flèche indique la crosse aortique sur l'image SPECT. L représente la gauche, R représente la droite, P représente la face postérieure.

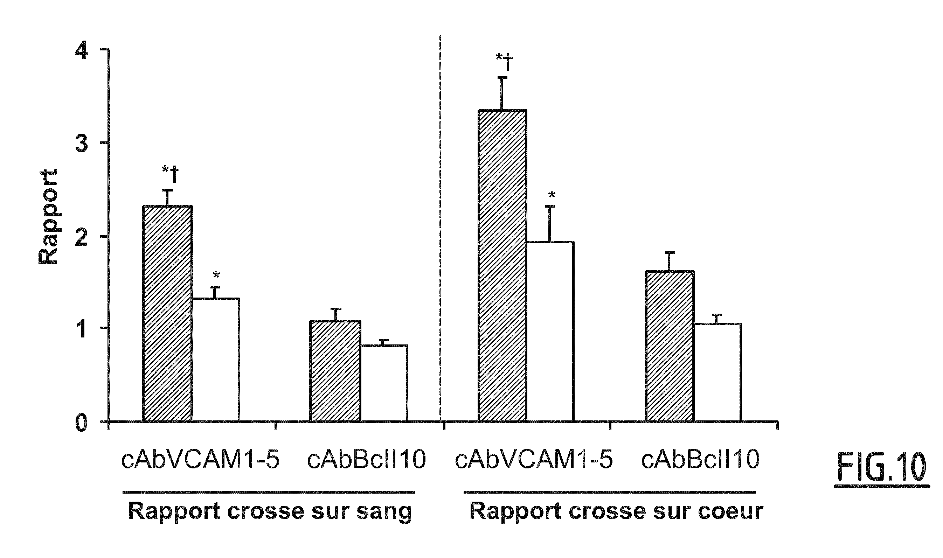

La **Figure 10** représente des histogrammes montrant les rapports *in vivo* crosse sur sang (panel de gauche) et crosse sur coeur (panel de droite) obtenus sur des souris contrôles C57BI/6J (barres blanches) ou sur des souris ApoE^{-/-} (barres noires) après administration de nanocorps contrôle cAbBcII10 ou de nanocorps cAbVCAM1-5, sur la base de quantifications d'images SPECT. * : P<0,05 *versus* ^{99m}Tc-AbBcII10. † : P<0,05 *versus* C57BI/6J.

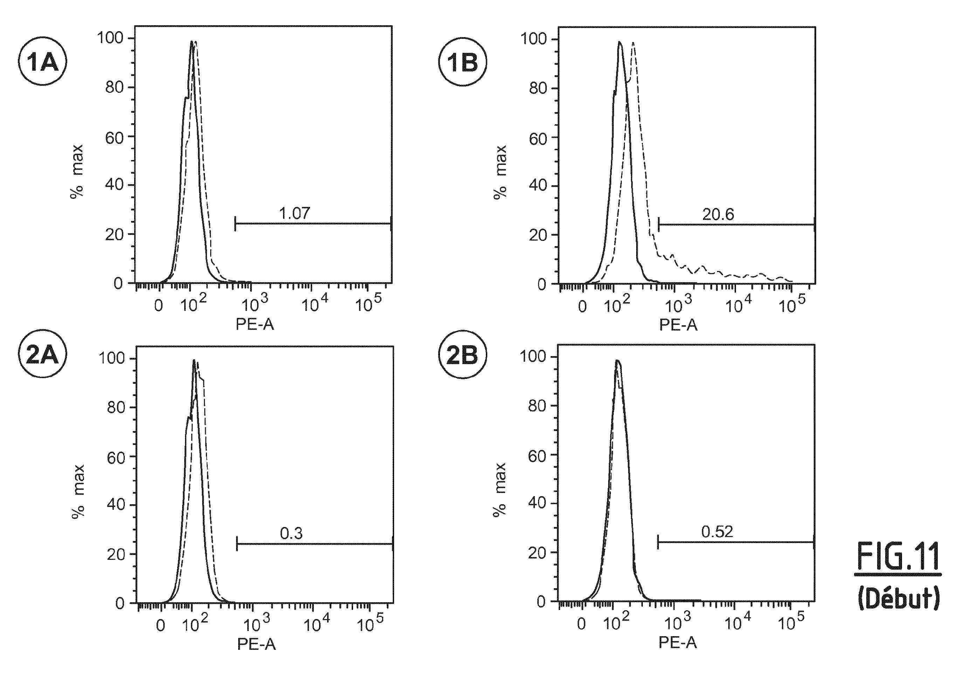

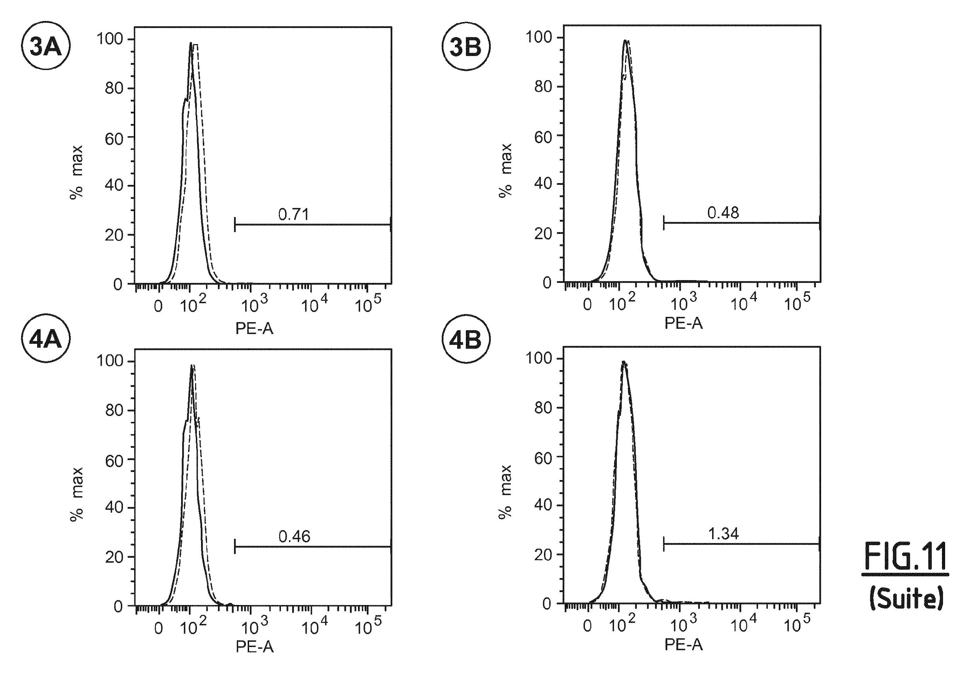

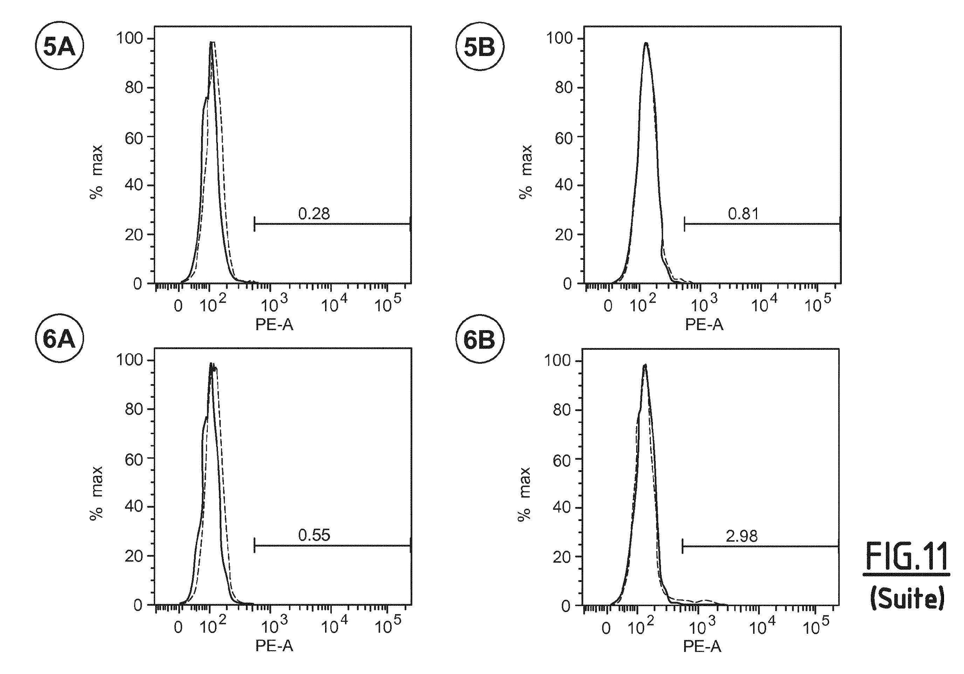

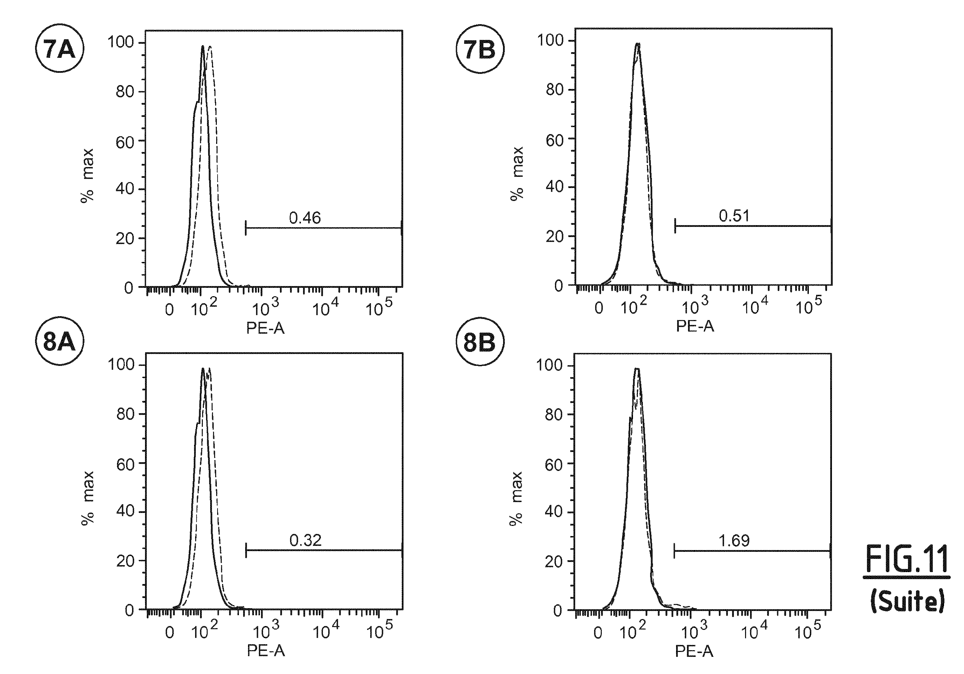

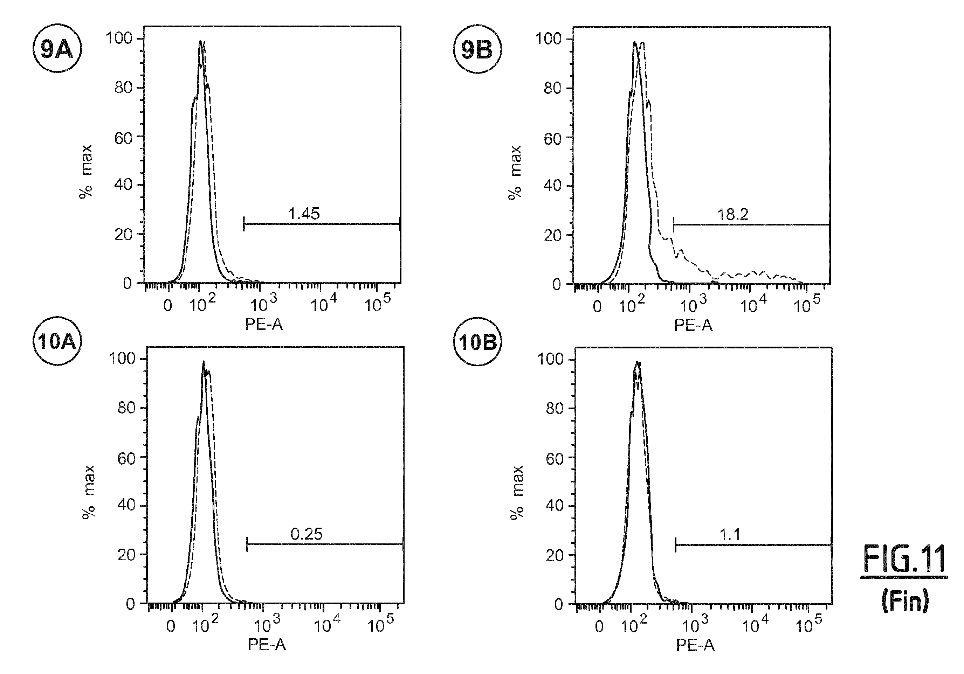

La **Figure 11** représente l'analyse par cytométrie en flux des nanocorps anti-VCAM-1 (cAbVCAM1-1 : 1; cAbVCAM1-2 : 2; cAbVCAM1-3 : 3; cAbVCAM1-4 : 4; cAbVCAM1-5 : 5; cAbVCAM1-6 : 6; cAbVCAM1-7 : 7; cAbVCAM1-8 : 8; cAbVCAM1-9 : 9 et cAbVCAM1-10: 10) (trait pointillé) sur des cellules de hamster CHO non transfectées (A) ou transfectées (B) avec un plasmide codant pour le VCAM-1 de lapin. Une condition sans nanocorps a été utilisée comme contrôle négatif (trait plein). Les nanocorps cAbVCAM1-1 et cAbVCAM1-5 se fixent aux cellules CHO exprimant le VCAM-1 de lapin.

### Exemple

L'exemple ci-dessous montre les propriétés spécifiques des nanocorps cAbVCAM1-5, cAbVCAM1-3, cAbVCAM1-8 et cAbVCAM1-9 selon l'invention par rapport à d'autres nanocorps anti-VCAM-1.

### Matériel et méthodes

### Génération et production de nanocorps

Des nanocorps ciblant VCAM-1 ont été générés de manière générale selon les méthodes précédemment publiées (Arbabi Ghahroudi et al. (1997) FEBS Lett. 414:521-526). Plus spécifiquement, un dromadaire a été immunisé avec des protéines VCAM-1 recombinantes à la fois humaines et murines (RnD Systems, Minneapolis, USA). Les lymphocytes du sang ont été isolés et l'ARN purifié. Les domaines variables des anticorps simple chaîne (VHH ou nanocorps) ont été amplifiés en utilisant la technique de la RT-PCR à deux étapes et clonés en phase avec la protéine 3 du bactériophage M13. Les nanocorps ont été soumis au phage-display et utilisés en biopanning sur des immunogènes immobilisés. Des extraits bactériens totaux contenant des nanocorps solubles ont été utilisés pour sélectionner des ligands de VCAM-1 individuels sur la base d'un signal positif en ELISA et en cytométrie en flux sur des cellules bEND5 stimulées avec TNFα. Après séquençage, les nanocorps anti-VCAM-1 sélectionnés et les nanocorps contrôles cAbBcII10 non pertinents ont été produits sous forme de protéines taggées avec une hexahistidine dans *E. coli* et purifiés comme décrit dans Saerens et al. (2005) J. Mol. Biol. 352:597-607.

### Evaluation in vitro de nanocorps non marqués

*Lignée cellulaire -* La lignée cellulaire endothéliale de souris bEND5 (ECACC, Salisburg, GB) a été cultivée dans du milieu DMEM complémenté. L'expression de VCAM-1 a été induite par stimulation avec 10 ng/mL de TNFα pendant 18 heures.

*Cytométrie en flux -* 10⁵ cellules bEND5 stimulées au TNFα et non-stimulées ont été incubées avec soit 200 ng d'anticorps monoclonal anti-VCAM-1 de souris marqué au PE (mAb; Abcam), soit séquentiellement avec 1 µg de nanocorps, 1 µg d'anticorps monoclonal anti-His-tag (Serotec) et 200 ng d'IgG₁ de rat anti-souris (BD Biosciences). La liaison a été mesurée sur un FACS Canto Il analyzer (BD Biosciences, Franklin Lakes, USA) et les données analysées avec le logiciel FlowJo (TreeStar, Ashland, USA).

*Stabilité thermique -* Les valeurs de Tₘ (températures de dépliement) ont été obtenues sur un spectropolarimètre J-715 (Jasco, Easton, MD, USA) comme décrit dans Vaneycken et al. (2010) J. Nucl. Med. 51:1099-1106.

*Evaluation de l'affinité basée sur la résonnance plasmonique de surface (SPR) -* L'affinité des nanocorps pour VCAM-1 recombinant humain ou murin a été déterminée par analyse SPR sur un appareil Biacore 3000. ICAM-1 de souris recombinant (RnD Systems) a été utilisé comme contrôle négatif. Les protéines recombinantes ont été immobilisées sur une puce capteur CM5 (Biacore, Uppsala, Suède) selon les instructions du fabricant. Des dilutions au demi en série des nanocorps de 50 à 1 nM dans du PBS 0,005% Tween-20^{®} ont été testées. Les constantes d'affinité ont été déterminées en utilisant un ajustement du modèle d'association standard 1:1 (logiciel BIAevaluation).

*Compétition avec l'épitope en utilisant la SPR -* La SPR a été utilisée pour déterminer quels nanocorps étaient en compétition pour le même épitope. Les procédures utilisées sont identiques à celles décrites dans Vaneycken et al. (2011) FASEB J. 25:2433-2446.

### Radiomarquage et détermination par HPLC de la stabilité in vitro et in vivo

Les nanocorps ont été radiomarqués avec ^{99m}Tc en utilisant la méthode tricarbonyle comme décrit dans Gainkam et al. (2008) J. Nucl. Med. 49:788-795. La pureté radiochimique a été évaluée immédiatement après le marquage, après 6 heures à 20°C dans du PBS et dans du sang de souris 3 h après injection (p.i.). Dans le dernier cas, 100 µL de sang total échantillonné ont été centrifugés et le plasma a été filtré en utilisant une membrane Nanosep 10K Omega. La pureté radiochimique a été déterminée par RP-HPLC en utilisant une colonne C4 éluée avec une phase mobile en gradient ACN/TFA. La radioactivité a été visualisée en utilisant un radiodétecteur (γ-RAM Model 4, LabLogic).

### Evaluation in vitro des nanocorps marqués au ^{99m}Tc

250×10³ cellules bEND5 ont été ensemencées dans des plaques 24-puits et stimulées pendant 18 heures avec du TNFα à 10 ng/mL. 5 nM de chaque nanocorps-^{99m}Tc ont été incubés dans 0,5 mL de PBS + 1% de sérum albumine humaine (HSA) pendant 1,5 heures à 37°C. Des études de compétition avec un excès de 500 fois de nanocorps non marqué ont été conduites pour déterminer la spécificité de liaison. Après lavage, les ^{99m}Tc-nanocorps liés ont été collectés et comptés dans un compteur gamma (Cobra Il Inspector 5003, Canberra Packard, USA). Les expériences ont été réalisées en triplicatas. La liaison non-spécifique aux puits a été soustraite, et les résultats ont été normalisés à la condition TNFα-négative.

### Modèle animal et traitement des aortes

Toutes les expériences animales ont été approuvées par le comité du Centre de Recherche du Service de Santé des Armées de Grenoble (CRSSA). Des souris femelles contrôles et ApoE^{-/-} âgées de 31±1 semaines ont été utilisées (Charles-River, France). Les souris ApoE^{-/-} (n=37) ont été nourries avec un régime occidental contenant 0,25% de cholestérol (Safe, France), alors que les souris C57BI/6J contrôles (n=9) ont été nourries avec un régime alimentaire standard. Chaque nanocorps anti-VCAM-1 a été évalué sur 3 souris ApoE^{-/-} sauf le nanocorps cAbVCAM1-5 (n=6), qui a été en outre évalué sur des souris C57BI/6J contrôles (n=4). L'anticorps cAbBcII10 a été évalué comme contrôle négatif à la fois dans les souris ApoE^{-/-} (n=4) et les souris contrôles (n=5). Deux heures après administration de nanocorps radiomarqués au ^{99m}Tc (67±4 MBq i.v.), les souris ont été anesthésiées en utilisant de l'isoflurane 2% (Baxter) et l'acquisition SPECT/CT a été réalisée (nanoSPECT, Bioscan, voir ci-dessous). Les souris ont été euthanasiées par une overdose de pentobarbital de sodium (CEVA) et les aortes, ainsi que les autres organes majeurs, ont été récupérées avec précaution. Les aortes ont été séparées des tissus adhérents dans une solution de formaline à 10% et coupées en 12 segments de l'aorte ascendante jusqu'à la bifurcation iliaque. Un index d'extension de la lésion a été attribué à chaque segment comme suit: (-) pas de lésion (segments contrôles), (+) lésions couvrant jusqu'à 50% de la longueur du segment artériel, (++) lésions couvrant plus de 50% de la longueur du segment artériel et (+++) lésions s'étendant sur toute la longueur du segment. Les segments aortiques et les échantillons de tissu ont été pesés et leur activité a été déterminée par un compteur-puit gamma (Cobra II, Packard). Les résultats ont été corrigés du bruit de fond et de la déccroissance radioactives et exprimés en pourcentage de la dose injectée par gramme de tissu (%ID/g). Les absorptions des lésions aortiques et du contrôle ont été définies comme l'absorption moyenne dans tous les segments classés (+++) ou (-) respectivement. Les rapports lésion sur contrôle, lésion sur sang et lésion sur coeur ont été aussi été déterminés.

Des cryo-sections adjacentes de 20 et 8 µm d'épaisseur ont été obtenues à partir des 12 segments aortiques obtenus pour l'imagerie par micro-autoradiographie (BASS-5000, Fujifilm) et le marquage immunohistologique de VCAM1, respectivement.

De plus, la clairance sanguine de ^{99m}TC-cAbVCAM1-5 a été évaluée dans 3 souris C57BI/6 par récupération d'échantillons sanguins à différents temps après injection. Les résultats ont été exprimés en %ID dans le volume de sang total (%ID/TBV).

### Immunohistochimie

Après 1 heure de blocage à 20°C avec 10% de sérum d'âne, un anticorps primaire de chèvre anti-VCAM-1 (Santa-Cruz Biotechnology, 0,5 µg/ml) a été appliqué sur les sections aortiques pendant la nuit à 4°C. Un anticorps secondaire biotinylé d'âne anti-chèvre (Jackson ImmunoResearch) a été incubé pendant 1 heure à 20°C et le DAB a été utilisé comme chromogène. Les sections ont été contre-colorées à l'hématoxyline. La spécificité de marquage a été vérifiée en omettant l'anticorps primaire sur des lames contrôles. Dans un sous-groupe de souris contrôles et ApoE^{-/-}, l'immunomarquage VCAM-1 a été réalisé sur le coeur, le muscle, les glandes salivaires, la moelle osseuse, les ganglions lymphatiques, la rate et le thymus.

### Imagerie SPECT/CT

Deux heures après les injections intraveineuses, les animaux anesthésiés ont été placés dans un lit à température contrôlée et les acquisitions SPECT/CT corps entier ont été réalisées (nanoSPECT, Bioscan). Le temps d'acquisition CT approximatif était de 10 min, en utilisant les paramètres d'acquisition suivants : 45kvp, 240 projections et 1000 ms par projection. L'acquisition SPECT hélicoïdale a été réalisée avec 4 têtes équipées avec des collimateurs sténopéiques à multi-trous de résolution 1 mm (9 × 1,4 mm de diamètre de trou par tête) en utilisant 24 projections et 45 minutes d'acquisition. Les acquisitions CT et SPECT ont été reconstruites, fusionnées et quantifiées en utilisant le logiciel dédié (InVivoScope). Les régions d'intérêt (ROIs) ont été dessinées au niveau de la crosse aortique, de la cavité ventriculaire gauche et de la paroi ventriculaire gauche pour déterminer les rapports crosse sur sang et crosse sur coeur.

### Autoradiographie

Pour chaque animal, les images autoradiographiques ont été obtenues après une nuit d'exposition de 3 groupes de lames de 20 µm d'épaisseur obtenus à différents niveaux des 12 segments aortiques. Les images ont été quantifiées en utilisant le logiciel dédié (Image Gauger, Fujifilm). Les ROIs ont été dessinées autour des lésions athérosclérotiques et de la paroi aortique VCAM-1 négative contrôle. Les résultats ont été corrigés pour le bruit de fond et exprimés sous forme de rapports lésion sur contrôle moyens.

### Evaluation de la réactivité des nanocorps non radiomarqués contre le VCAM-1 de lapin.

10⁵ cellules CHO transfectées ou non de manière transitoire avec un plasmide codant pour le VCAM-1 de lapin ont été incubées séquentiellement avec 1 µg de nanocorps, 1 µg d'anticorps monoclonal anti-His-tag (Serotec) et 200 ng d'IgG₁ de rat anti-souris (BD Biosciences). La liaison a été mesurée sur un FACS Canto Il analyzer (BD Biosciences, Franklin Lakes, USA) et les données analysées avec le logiciel FlowJo (TreeStar, Ashland, USA). Un contrôle négatif a été réalisé en omettant le nanocorps.

### Analyse statistique

Tous les résultats sont présentés sous la forme de moyenne ± erreur standard de la moyenne. Les tests non-paramétriques de Mann&Witney U et de Wilcoxon ont été utilisés pour comparer des données appariées et non-appariées. Les différences ont été considérées comme significatives pour p<0,05.

### Résultats

### Génération des nanocorps anti-VCAM-1

Les inventeurs ont cherché à développer des nanocorps à réaction croisée pour VCAM-1 humain et murin. Par conséquent, les nanocorps ont été générés par immunisation d'un dromadaire avec des protéines recombinantes VCAM-1 à la fois humaines et murines et par biopanning de la bibliothèque de nanocorps immuns obtenus par phage-display en résultant. Des extraits bactériens totaux contenant les nanocorps individuels ont été criblés en ELISA pour leur liaison avec les protéines recombinantes VCAM-1 et en cytométrie de flux pour leur liaison aux cellules bEND5 exprimant VCAM-1.

Après séquençage, 31 nanocorps anti-VCAM-1 ont été identifiés et regroupés en 12 familles sur la base de la similarité des séquences dans les boucles de liaison à l'antigène. Six familles de nanocorps étaient spécifiques de VCAM-1 murin (mVCAM-1) et 6 familles se liaient à la fois au VCAM-1 murin et humain (hVCAM-1). Sur la base des signaux ELISA et de cytométrie en flux des extraits totaux, 10 nanocorps (appelés cAbVCAM1-1 à -10) ont été sélectionnés pour être analysés plus en détail. Le rendement de production de nanocorps était de 0,5 à 10,5 mg/L de culture bactérienne **(Tableau 4).**

### Caractérisations in vitro

Les expériences de cytométrie en flux sur les cellules bEND5 ont montré que les 10 nanocorps sélectionnés interagissaient avec VCAM-1 **(****Figure 1****).** Comme démontré par les analyses SPR, résumées dans le **Tableau 4,** tous les nanocorps sélectionnés se liaient à VCAM-1 avec de hautes affinités allant de 0,2 à 45,7 nM. De plus, parmi les 10 nanocorps sélectionnés, 6 étaient cross-réactifs avec hVCAM-1 avec des affinités restant de l'ordre du nanomolaire **(Tableau 4).** Ces six nanocorps étaient cAbVCAM1-1, cAbVCAM1-3, cAbVCAM1-5, cAbVCAM1-8, cAbVCAM1-9 et cAbVCAM1-10. Sur la base d'études de compétition SPR, les nanocorps cAbVCAM1 ont été groupés en 3 catégories de ciblage d'épitope : cAbVCAMI-1/5, cAbVCAM1-2/3/6/7/9/10 et cAbVCAM1-4/8. Tous les nanocorps ont montré une forte stabilité thermique comme démontré par les températures de dépliement allant de 59,4 à plus de 87°C (**Tableau 4).**

**Tableau 4: Comparaison entre les 10 nanocorps anti-VCAM-1 évalués.**

| **Radiotraceur** | **%ID/g de lésion** | **Lésion/Contrôle** | **Lésion/Sang** | **Lésion/Coeur** | **K_{D} mVCAM-1 (nM)** | **K_{D} hVCAM-1 (nM)** | **Rendement de production (mg/L)** | **Tₘ (°C)** |
|---|---|---|---|---|---|---|---|---|
| **cAbVCAM1-1** | 0,87±0,08 #9 | 2,15±0,20 #9 | 0,74±0,10* #10 | 2,65±0,23* #9 | 8,3±1,2 #7 | 12,4±0,5 #5 | 2,0 #7 | 72,3±0,1 #2 |
| **cAbVCAM1-2** | 2,15±0,29* #6 | 2,90±0,45 #2 | 3,37±0,32* #5 | 5,55±0,58* #7 | 0,3±0,0 #2 | Non cross-réactif | 5,0 #5 | 62,3±0,1 #6 |
| **cAbVCAM1-3** | 2,95±0,16* #2 | 4,07±0,56 #3 | 5,06±0,39* #1 | 7,40±0,91* #3 | 2,4±0,1 #5 | 9,1±0,9 #4 | 6,8 #3 | 59,7±0,1 #9 |
| **cAbVCAM1-4** | 2,21±0,59* #5 | 3,20±0,74 #5 | 1,41±0,29 #9 | 1,96±0,56* #10 | 0,2±0,0 #1 | Non cross-réactif | 6,8 #3 | 59,4±0,1 #10 |
| **cAbVCAM1-5** | 2,53±0,08* #3 | 4,95±0,85* #1 | 4,32±0,48* #2 | 8,30±1,11* #1 | 2,0±0,0 #4 | 6,5±0,7 #3 | 10,5 #1 | >87 #1 |
| **cAbVCAM1-6** | 0,73±0,08 #10 | 4,57±0,93* #2 | 1,85±0,37 #8 | 4,98±0,75 #8 | 5,2±0,6 #6 | Non cross-réactif | 3,0 #6 | 72,0±0,1 #3 |
| **cAbVCAM1-7** | 1,27±0,25* #8 | 2,88±0,65 #7 | 4,02±1,05* #3 | 5,98±0,96 #4 | 26,6±1,2 #9 | Non cross-réactif | 6,9 #2 | 60,9±0,3 #8 |
| **cAbVCAM1-8** | 2,48±0,46* #4 | 1,40±0,10 #10 | 3,66±0,10* #4 | 7,71±0,38* #2 | 13,.2±0,3 #8 | 1,4±0,5 #1 | 1,5 #8 | 61,5±0,1 #7 |
| **cAbVCAM1-9** | 2,99±0,07* #1 | 2,19±0,60 #8 | 2,51±0,03* #6 | 5,69±0,36* #6 | 0,9±0,2 #3 | 5,3±0,7 #2 | 0,9 #9 | 66,8±0,2 #4 |
| **cAbVCAM1-10** | 1,93±0,14* #7 | 3,47±0,67* #4 | 2,01±0,14* #7 | 5,76±0,56* #5 | 45,7±20,0 #10 | 18,4±7,0 #6 | 0,8 #10 | 63,4±0,2 #5 |
| **cAbBcII10** | 0,68±0,06 | 1,66±0,28 | 1,57±0,09 | 4,00±0,14 | - | - | - | - |

| | | | | | | | | |
|---|---|---|---|---|---|---|---|---|
| La moyenne ±erreur standard de la moyenne et le rang (#) sont donnés pour les paramètres obtenus soit *ex vivo* par comptage-puit gamma (%ID/g de lésion, rapport lésion sur contrôle, rapport lésion sur sang, rapport lésion sur coeur), ou *in vitro* (K_{D} pour mVCAM-1 ou hVCAM-1, rendement de production et Tₘ). * p<0,05 versus cAbBcII10. | | | | | | | | |

Suite aux étapes de radiomarquage au ^{99m}Tc et de purification, les puretés radiochimiques étaient supérieures à 95% pour les 10 nanocorps cAbVCAM1 et le nanocorps contrôle cAbBcII10. Le marquage au ^{99m}Tc n'a pas affecté la reconnaissance de VCAM-1 pour la plupart des ligands, comme démontré par le test de liaison *in vitro* sur les cellules bEND5 **(****Figure 2****) :** les inventeurs ont en effet montré que la liaison aux cellules endothéliales VCAM-1 positives et stimulées au TNFα était significativement plus forte que sur les cellules stimulées. De plus, la liaison aux cellules stimulées au TNFα a été inhibée avec succès par compétition avec un excès de nanocorps non marqué, ce qui démontre leur spécificité.

### Analyses des biodistributions

Les biodistributions des nanocorps marqués au ^{99m}Tc dans les souris ApoE^{-/-} sont résumées dans le **Tableau 5.** Tous les nanocorps, y compris le contrôle cAbBcII10, ont montré une forte absorption au niveau des reins, allant de 97±16 à 315±33 %ID/g et de fortes activités dans la vessie. De manière intéressante, les absorptions de ^{99m}Tc-cAbVCAM1 étaient élevées dans les tissus lymphoïdes et ont même atteint une différence statistique pour ^{99m}Tc-cAbVCAM1-3 (rate et thymus), ^{99m}Tc-cAbVCAM1-4/5 (rate, thymus et moelle osseuse) et ^{99m}Tc-cAbVCAM1-9 (thymus) (p<0,05 *versus* ^{99m}Tc-cAbBcII10). A l'exception des poumons et du foie (absorption moyenne de 2,5±0,8 et 2,7±0,9 %ID/g, respectivement), l'absorption était inférieure à 2 %ID/g dans les autres tissus étudiés, y compris le sang et le myocarde.

Comme montré dans le **Tableau 4,** l'absorption dans les lésions athérosclérotiques était supérieure à 2 %ID/g pour 6 des 10 nanocorps cAbVCAM1, avec une valeur maximum de 2,99±0,14 %ID/g pour ^{99m}Tc-cAbVCAM1-9 (P<0,05 *versus* ^{99m}Tc-cAbBcII10).

Les rapports lésion sur contrôle, lésion sur sang et lésion sur coeur ont été déterminés à partir des données de biodistribution **(Tableau 4).** Le rapport lésion sur contrôle était supérieur à 2 pour tous les nanocorps cAbVCAM1 évalués à l'exception d'un, avec un rapport maximum de 4,95±0,85 pour cAbVCAM1-5 (P<0,05 *versus* ^{99m}Tc-cAbBcII10). Le rapport lésion sur sang était supérieur à 1 pour tous les nanocorps cAbVCAM1 évalués à l'exception d'un, avec un rapport maximum de 5,6±0,39 pour ^{99m}Tc-cAbVCAM1-3 (P<0,05 *versus* ^{99m}Tc-cAbBcII10). Enfin, le ratio lésion sur coeur était supérieur à 1 pour tous les nanocorps, avec une valeur maximale de 8,30±1,11 pour ^{99m}Tc-cAbVCAM1-5 (P<0,05 *versus* ^{99m}Tc-cAbBcII10).

**Tableau 5: Biodistribution ex vivo des nanocorps anti-VCAM-1 marqués au ^{99m}Tc 3 heures après injection dans des souris hypercholesterolémiques ApoE^{-/-}**

| | **ApoE^{-/-}** | | | | | | | | | | |
|---|---|---|---|---|---|---|---|---|---|---|---|
| | **cAbVCAM1-** | | | | | | | | | | **cAbBcII10** |
| | **1** | **2** | **3** | **4** | **5** | **6** | **7** | **8** | **9** | **10** | |
| **Sang** | 1,2±0,1* | 0,6±0,1 | 0,6±0,0 | 1,5±0,1* | 0,6±0,1 | 0,4±0,1 | 0,3±0,0 | 0,6±0,1 | 1,2±0,0* | 1,0±0,2* | 0,4±0,0 |
| **Coeur** | 0,3±0,0 | 0,4±0,1* | 0,4±0,0* | 1,1±0,0* | 0,3±0,1* | 0,2±0,0 | 0,2±0,0 | 0,3±0,1 | 0,5±0,0* | 0,4±0,1 | 0,2±0,0 |
| **Poumon** | 3,2±0,6 | 1,5±0,2 | 3,2±0,5 | 4,1±1,0* | 2,3±0,3* | 1,7±1,1 | 1,3±0,2 | 2,1±0,6 | 2,5±0,1 | 2,07±1,0 | 1,0±0,2 |
| **Foie** | 2,2±0,24* | 2,0±0,1 | 1,5±0,1 | 8,3±0,7* | 1,8±0,3* | 0,8±0,1 | 1,2±0,2 | 3,1±0,3* | 2,4±0,2* | 4,0±0,4* | 0,7±0,0 |
| **Muscle squelettique** | 0,6±0,4 | 0,1±0,0 | 0,2±0,0 | 0,2±0,0 | 0,2±0,0 | 0,2±0,1 | 0,1±0,0 | 0,2±0,1 | 0,2±0,0 | 0,2±0,0 | 0,1±0,0 |
| **Peau** | 0,2±0,1 | 0,3±0,1 | 0,3±0,0 | 0,9±0,5 | 0,4±0,0 | 0,3±0,0 | 0,4±0,0 | 0,6±0,2 | 0,4±0,1 | 0,4±0,1 | 0,3±0,0 |
| **Graisse** | 0,1±0,0 | 0,1±0,0 | 0,1±0,0 | 0,2±0,0 | 0,1±0,0* | 0,1±0,0 | 0,1±0,0 | 0,2±0,1 | 0,1±0,0 | 0,1±0,0 * | 0,1±0,0 |
| **Cerveau** | 0,0±0,0* | 0,1±0,0* | 0,1±0,0* | 0,2±0,0 | 0,1±0,0* | 0,0±0,0 | 0,0±0,0 | 0,0±0,0 | 0,1±0,0* | 0,0±0,0* | 0,0±0,0 |
| **Glandes salivaires** | 0,9±0,3* | 0,4±0,0 | 0,5±0,0 | 1,2±0,1* | 0,5±0,1* | 0,3±0,0 | 0,5±0,1 | 0,6±0,3 | 0,6±0,1 | 0,7±0,1 | 0,2±0,0 |
| **Pancréas** | 0,3±0,1 | 0,2±0,0 | 0,3±0,0 | 0,6±0,0* | 0,3±0,0* | 0,2±0,0 | 0,2±0,0 | 0,3±0,1 | 0,4±0,0* | 0,3±0,1 | 0,1±0,0 |
| **Thyroïde** | 0,9±0,2 | 0,5±0,2 | 1,0±0,2 | 1,5±0,2 | 0,7±0,1 | 1,0±0,6 | 0,9±0,2 | 1,1±0,4 | 1,3±0,5 | 1,4±0,1 | 0,1±0,0 |
| **Estomac** | 0,6±0,0 | 0,6±0,1 | 0,6±0,0 | 1,5±0,3* | 0,6±0,1* | 0,3±0,0 | 0,5±0,1 | 0,7±0,1 | 0,7±0,0 | 0,7±0,1 | 0,4±0,1 |
| **Bile** | 0,9±0,2 | 0,4±0,1 | 1,7±1,0 | 0,5±0,0 | 0,7±0,2 | 0,5±0,1 | 0,9±0,1 | 1,1±0,1 | --- | 0,8±0,2 | 0,4±0,1 |
| **Intestin** | 1,4±0,8 | 0,5±0,1 | 0,5±0,0 | 0,7±0,4* | 0,5±0,1* | 0,3±0,1 | 0,6±0, | 0, 5±0,1 | 0,6±0,0 | 0,5±0,1 | 0,2±0,1 |
| **Rein** | 125±13 | 228±34 | 303±68 | 97±16* | 222±12 | 207±31 | 254±18 | 315±33 | 158±6 | 304±32 | 266±14 |
| **Urine** | 265±84* | 59±14 | 96±35 | 58±33 | 83±15 | 77±18 | 92±25 | 27±7 | 98±12 | 44±14 | 41±10 |
| **Rate** | 2,9±0,2 | 8,0±0,3 | 20,3±11,4* | 35,7±0,3* | 9,2±1,0* | 1,7±0,0 | 1,6±0,1 | 1,6±0,2 | 8,0±1,2 | 4,3±0,5 | 0,4±0,0 |
| **Thymus** | 0,4±0,1 | 0,7±0,1 | 1,9±0,1* | 2,2±0,6* | 1,7±0,1* | 0,4±0,0 | 0,5±0,0 | 0,5±0,1 | 1,5±0,2* | 0,3±0,2 | 0,2±0,0 |
| **Moelle osseuse** | 2,6±0,3 | 5,9±0,8 | 13,7±5,6 | 31,8±2,2* | 10,7±2,9* | 1,9±0,2 | 2,6±0,1 | 1,5±0,3 | 7,0±0,8 | 3,2±0,7 | 1,0±0,7 |

| | | | | | | | | | | | |
|---|---|---|---|---|---|---|---|---|---|---|---|
| Les résultats sont exprimés en moyenne ± erreur standard de la moyenne. * : p<0,05 *versus* cAbBcII10. | | | | | | | | | | | |

### Immunohistochimie

Comme montré dans la **Figure 3****,** une expression constitutive de VCAM-1 a été observée dans les tissus lymphoïdes (*i.e.* la moelle osseuse, les ganglions lymphatiques, la rate et le thymus) à la fois chez les souris contrôles C57BI/6J et les souris hypercholesterolémiques ApoE^{-/-}, tandis qu'aucune expression de VCAM1 n'a été trouvée dans le coeur, les muscles et les glandes salivaires. De plus, un fort marquage VCAM-1 a également été observé dans les lésions aortiques, au niveau de l'endothélium, ainsi qu'à l'intérieur de la plaque athérosclérotique, mais pas dans l'aorte des souris contrôles C57BI/6J.

### Absorption de cAbVCAM1-5 dans les lésions athérosclérotiques : imagerie ex vivo et in vivo

Sur la base des critères de sélection résumés dans le **Tableau 4,** les nanocorps cAbVCAM1-5, cAbVCAM1-3, cAbVCAM1-8 et cAbVCAM1-9 ont été considérés par les inventeurs comme les plus promoteurs pour servir de radiotraceurs en imagerie médicale. Le nanocorps cAbVCAM1-5 a été plus particulièrement sélectionné pour faire l'objet d'évaluations supplémentaires.

*Absorption aortique -* Dans les souris ApoE^{-/-}, les inventeurs ont montré que l'absorption aortique de ^{99m}Tc-cAbVCAM1-5 était en accord avec l'index d'extension de la lésion. En effet, l'absorption de ^{99m}Tc-cAbVCAM1-5 a augmenté en même temps que le volume relatif de la lésion athérosclérotique, tandis qu'un tel gradient n'a pas été observé avec ^{99m}Tc-cAbBcII10 **(****Figure 4****).** L'absorption de ^{99m}Tc-cAbVCAM1-5 dans l'aorte a de plus été caractérisée par autoradiographie. Comme montré dans la **Figure 5****,** ^{99m}Tc-cAbVCAM1-5 s'est accumulé au niveau des lésions athérosclérotiques positives à VCAM-1, entraînant un rapport lésion sur contrôle de 8,7±0,5 (P<0,05 *versus* cAbBcII10).

*Stabilité -* Les inventeurs ont démontré par HPLC que ^{99m}Tc-cAbVCAM1-5 était stable *in vitro* jusqu'à 6 heures après le radiomarquage, et *in vivo* dans le sang 3 heures après injection, au moment de l'imagerie SPECT **(****Figure 6****).**

*Biodistribution dans les souris contrôles -* ^{99m}Tc-cAbVCAM1-5 a rapidement été éliminé de la circulation et une absorption dans les reins, la vessie et les tissus lymphoïdes a été clairement identifiée sur les images SPECT *in vivo* à la fois chez les souris contrôles C57BI/6J et les souris ApoE^{-/-} athérosclérotiques, alors que seuls les reins et la vessie étaient visibles après injection de ^{99m}Tc-cAbBcII10 **(****Figure 7****).** L'absorption de ^{99m}Tc-cAbVCAM1-5 dans les tissus lymphoïdes des souris contrôles C57BI/6J a de plus été confirmée *ex vivo* par des analyses de biodistribution **(Tableau 6).**

**Tableau 6 : Biodistribution ex vivo des nanocorps cAbVCAM1-5 et cAbBcII10 marqués au ^{99m}Tc 3 heures après injection à des souris contrôles C57BI/6J.**

| | **C57BI/6J** | |
|---|---|---|
| | **cAbVCAM1-5** | **cAbBcII10** |
| **Sang** | 0,5±0,1 | 0,4±0,0 |
| **Coeur** | 0,2±0,0* | 0,1±0,0 |
| **Poumon** | 1,7±0,2* | 0,8±0,2 |
| **Foie** | 1,4±0,2 | 1,0±0,1 |
| **Muscle squelettique** | 0,1±0,0 | 0,1±0,0 |
| **Peau** | 0,4±0,0 | 0,4±0,0 |
| **Graisse** | 0,2±0,0 | 0,1±0,0 |
| **Cerveau** | 0,0±0,0* | 0,0±0,0 |
| **Glande salivaire** | 0,5±0,0* | 0,3±0,0 |
| **Pancréas** | 0,2±0,0 | 0,2±0,0 |
| **Thyroïde** | 0,7±0,1 | 0,5±0,1 |
| **Estomac** | 0,5±0,0* | 0,4±0,0 |
| **Bile** | 0,3±0,0 | 0,5±0,1 |
| **Intestin** | 0,5±0,1 | 0,3±0,0 |
| **Rein** | 287±43 | 350±16 |
| **Urine** | 59±23 | 47±8 |
| **Rate** | 7,4±0,2* | 0,3±0,0 |
| **Thymus** | 1,5±0,1* | 0,1±0,0 |
| **Moelle osseuse** | 7,9±2,0* | 0,4±0,0 |

| | | |
|---|---|---|
| Les résultats sont présentés en moyenne ± erreur standard de la moyenne. * : P<0,05 *versus* cAbBcII10. | | |

En effet, les inventeurs ont montré que l'absorption de ^{99m}Tc-cAbVCAM1-5 représentait 7,4±0,2, 1,5±0,1 et 7,9±2,0 %ID/g dans la rate, le thymus et la moelle osseuse, respectivement (P<0,05 *versus* ^{99m}Tc-cAbBcII10 dans les trois tissus).

*Imagerie SPECT*/*CT -* Suite à l'imagerie SPECT/CT, l'absorption de ^{99m}Tc-cAbVCAM1-5 a été visualisée sur les lésions athérosclérotiques de la crosse aortique de souris ApoE^{-/-}, alors qu'aucune absorption de traceur n'a été observée au même endroit chez les animaux C57BI/6J **(****Figures 8** **et** **9****).** Par conséquent, les rapports crosse sur sang et crosse sur coeur se sont révélés significativement plus élevés chez les souris ApoE^{-/-} par rapport aux souris C57BI/6J et par rapport au nanocorps contrôle négatif (P<0,05; **Figure 10****).**

*Réactivité des nanocorps non radiomarqués contre le VCAM-1 de lapin -* Les inventeurs ont démontré par cytométrie en flux que cAbVCAM1-1 et cAbVCAM1-5 se liaient spécifiquement au VCAM-1 de lapin **(****Figure 11****).**

### Discussion

Cette étude a été mise au point par les inventeurs afin de générer des nanocorps reconnaissant à la fois les homologues VCAM-1 humain et murin, dans la mesure où de tels ligands cross-réactifs sont particulièrement intéressants pour transposer à la pratique clinique les résultats validés en modèles animaux bien caractérisés. Dix nanocorps anti-VCAM-1 ont été générés et produits avec des affinités pour les homologues murin et/ou humain de l'odre du nanomolaire, parmi lesquels 6 nanocorps cross-réactifs. De plus deux nanocorps, cAbVCAM1-1 et cAbVCAM1-5, sont cross réactifs pour le VCAM-1 de lapin. La résistance thermique élevée des nanocorps, en particulier de cAbVCAM1-5, a permis leur radiomarquage au ^{99m}Tc à 50°C avec une haute pureté radiochimique (> 95%). De plus, les tests de liaison *in vitro* sur des cellules endothéliales de souris VCAM-1 positives a révélé que ces nanocorps marqués restaient des ligands spécifiques de mVCAM1.

Du fait de leur faible taille, les nanocorps présentaient une clairance sanguine rapide *in vivo,* résultant en une activité circulatoire moyenne de 0,8% ID/g 3 heures après injection dans des souris Apo^{-/-}. De plus, l'activité bruit de fond au niveau du myocarde était également minimale. De manière importante, l'absorption des nanocorps anti-VCAM-1 au niveau des lésions aortiques était significativement plus élevée que celle du nanocorps contrôle négatif cAbBcII10, en particulier pour les nanocorps cAbVCAM1-5 et cAbVCAM1-3. Par conséquent, les rapports lésion sur contrôle, lésion sur sang et lésion sur coeur étaient supérieurs à 1, en particulier pour les nanocorps cAbVCAM1-5, cAbVCAM1-3, cAbVCAM1-8 et cAbVCAM1-9.

### Absorption dans les tissus lymphoïdes

En plus d'être absorbés dans les lésions athérosclérotiques, les nanocorps selon l'invention ont également été absorbés par les tissus lymphoïdes, à la fois chez les souris normales et hypercholestérolémiques, comme démontré par les expériences de biodistribution et d'imagerie SPECT. En particulier les trois nanocorps cross-réactifs avec les plus fortes affinités pour mVCAM-1 (cAbVCAM1-5/3/9) ont montré les plus fortes absorptions dans la rate et la moelle osseuse. L'expression constituve de mVCAM-1 a été observée par immunohistochimie dans la rate, la moelle osseuse, les ganglions lymphatiques et le thymus. Par conséquent, la liaison des nanocorps anti-VCAM-1 aux tissus lymphoïdes était probablement due à leur liaison spécifique à la protéine VCAM-1.

### Sélection de composés d'intérêt particulier

Sur la base des paramètres résumés dans le **Tableau 4,** les nanocorps cAbVCAM1-5, cAbVCAM1-3, cAbVCAM1-8 et cAbVCAM1-9 ont été identifiés par les inventeurs comme ayant des propriétés particulièrement avantageuses par rapport aux autres nanocorps, en particulier en vue d'une utilisation en imagerie médicale. Le nanocorps le plus prometteur est le nanocorps cAbVCAM1-5. En effet, cAbVCAM1-5 présente les rapports lésion sur contrôle et lésion sur coeur les plus élevés, ainsi qu'un fort rapport lésion sur sang. Il présente de surcroît une bonne affinité à la fois pour VCAM-1 humain et murin et à la plus haute résistance thermique et le rendement de production le plus élevé. Enfin, cAbVCAM1-5 est cross réactif pour le VCAM-1 de lapin, un modèle animal de référence dans l'étude de l'athérosclérose. cAbVCAM1-3 présente le rapport lésion sur sang le plus élevé et de forts rapports lésion sur contrôle et lésion sur coeur. cAbVCAM1-8 présente un rapport lésion sur coeur particulièrement élevé et la meilleure affinité pour VCAM-1 humain. Enfin, cAbVCAM1-9 présente la meilleure incorporation au niveau des lésions athérosclérotiques et de très bonnes affinités pour VCAM-1 humain et murin. De surcroît, AbVCAM1-5, AbVCAM1-3 et AbVCAM1-9 n'ont pas de lysine au niveau de leurs CDRs, ce qui est un avantage dans la mesure où la présence de lysine pourrait présenter un inconvénient pour les techniques de couplage permettant de marquer les nanocorps par fluorescence ou par radiomarquage, via leurs résidus amines, en vue d'imagerie PET.

### Imagerie in vivo de cAbVCAM1-5

^{99m}Tc-cAbVCAM1-5 est stable *in vitro* jusqu'à 6 heures après le radiomarquage ainsi qu'*in vivo* dans le sang de souris, comme démontré par HPLC. Ceci permet de mettre en oeuvre l'imagerie SPECT/CT 3 heures après injection. A ce moment, les lésions athérosclérotiques situées au niveau de la crosse aortique de souris ApoE^{-/-} ont été identifiées avec succès par les inventeurs par imagerie SPECT/CT, avec de faibles activités bruit de fond au niveau du myocarde et du sang. L'autoradioradiographie et l'immunohistochimie ont confirmé que l'absorption aortique de ^{99m}Tc-cAbVCAM1-5 était focalisée dans les lésions athérosclérotiques VCAM-1 positives. Ces expériences montrent donc que ^{99m}Tc-cAbVCAM1-5 est un radiotraceur approprié pour l'imagerie *in vivo* non invasive de processus inflammatoires ayant lieu au niveau des lésions athérosclérotiques.

### Comparaison avec d'autres radiotraceurs

D'autres radiotraceurs dérivés d'anticorps ont été récemment évalués pour l'imagerie de plaques d'athérome vulnérables en utilisant la SPECT (Temma et al. (2010) J. Nucl. Med. 51:1979-1986; Kuge et al. (2010) Eur. J. Nucl. Med. Mol. Imaging 37:2093-2104). Cependant, la faible clairance des anticorps entiers entraîne des rapports cible sur bruit de fond suboptimaux, soulignant le besoin d'utiliser des fragments d'anticorps. Parmi les autres radiotraceurs précédemment évalués pour l'imagerie SPECT ou PET des lésions athérosclérotiques, le ¹⁸FDG a montré une absorption élevée dans les macrophages, permettant l'imagerie *in vivo* des lésions carotidiennes chez l'homme (Rudd et al. (2002) Circulation 105:2708-2711). Cependant, du fait du fort bruit de fond au niveau du myocarde, l'imagerie des lésions coronariennes reste très difficile malgré l'utilisation potentielle d'un régime spécifique destiné à diminuer l'absorption au niveau du myocarde (Wykrzykowska et al. (2009) J. Nucl. Med. 50:563-568). De manière similaire, dans un modèle souris d'athérosclérose, Laitinen *et al.* ont montré que l'absorption au niveau du myocarde de ¹⁸FDG était de 18,13±10,59 %ID/g par rapport à 0,41±0,16 %ID/g dans les lésions athérosclérotiques 1 heure après injection (Laitinen et al. (2006) Eur. J. Nucl. Med. Mol. Imaging 33:1461-1467).

### Conclusion

Les présents inventeurs ont ainsi identifiés et produits quatre nanocorps anti-VCAM-1, cross-réactifs pour VCAM-1 humain, présentant des caractéristiques importantes pour une utilisation en imagerie médicale. Les inventeurs ont en particulier directement montré que ^{99m}Tc-cAbVCAM1-5 permettait d'identifier avec succès des lésions athérosclérotiques *in vivo* par imagerie SPECT/CT.

## Revendications

1. Nanocorps dirigé contre VCAM-1 comprenant :
a) les séquences d'acides aminés (i) YTNSIMYMA (SEQ ID NO: 1) comme CDR1, (ii) AIRFPDDS (SEQ ID NO: 2) comme CDR2 et (iii) RSSPYSFAWNDPSNYNY (SEQ ID NO: 3) comme CDR3 ; ou
b) les séquences d'acides aminés (i) FTYSSYYMS (SEQ ID NO: 4) comme CDR1, (ii) GINVDGSN (SEQ ID NO: 5) comme CDR2 et (iii) GSGRDSYDCYSGSWCP (SEQ ID NO: 6) comme CDR3 ; ou
c) les séquences d'acides aminés (i) FTFSNYYMT (SEQ ID NO: 7) comme CDR1, (ii) RINSDGS (SEQ ID NO: 8) comme CDR2 et (iii) GKSSV (SEQ ID NO: 9) comme CDR3 ; ou
d) les séquences d'acides aminés (i) FTFSSYYMS (SEQ ID NO: 10) comme CDR1, (ii) GINVDGSN (SEQ ID NO: 11) comme CDR2 et (iii) GSGRDSYDCYSGSWCP (SEQ ID NO: 12) comme CDR3 ;
ou un variant fonctionnellement conservatif du nanocorps défini en a), b), c) ou d) comprenant une substitution conservative d'un ou deux acides aminés dans respectivement une, deux ou trois des séquences SEQ ID NO: 1, SEQ ID NO: 2 et SEQ ID NO: 3, ou SEQ ID NO: 4, SEQ ID NO: 5 et SEQ ID NO: 6, ou SEQ ID NO: 7, SEQ ID NO: 8 et SEQ ID NO: 9, ou SEQ ID NO: 10, SEQ ID NO: 11 et SEQ ID NO: 12.

2. Nanocorps selon la revendication 1, ledit nanocorps comprenant une séquence d'acides aminés sélectionnée dans le groupe consistant en les séquences d'acides aminés SEQ ID NO: 13, SEQ ID NO: 14, SEQ ID NO: 15 et SEQ ID NO: 16.

3. Nanocorps selon la revendication 1 ou 2, ledit nanocorps consistant en la séquence d'acides aminés SEQ ID NO: 13.

4. Nanocorps selon l'une quelconque des revendications 1 à 3, ledit nanocorps étant associé à un marqueur détectable.

5. Nanocorps selon la revendication 4, ledit marqueur détectable étant un radioélément.

6. Nanocorps selon l'une quelconque des revendications 1 à 5 pour son utilisation comme agent de contraste dans l'imagerie médicale *in vivo,* non invasive.

7. Nanocorps selon l'une quelconque des revendications 1 à 6, pour son utilisation dans des méthodes de diagnostic ou de pronostic.

8. Nanocorps selon l'une quelconque des revendications 1 à 3 pour son utilisation comme médicament.

9. Utilisation d'un nanocorps tel que défini dans l'une quelconque des revendications 1 à 5 pour la détection *in vitro* de VCAM-1 dans un échantillon.

10. Composition pharmaceutique comprenant un nanocorps tel que défini dans l'une quelconque des revendications 1 à 3 en association avec un véhicule pharmaceutiquement acceptable.

11. Acide nucléique comprenant une séquence nucléique codant le nanocorps tel que défini dans l'une quelconque des revendications 1 à 3.

12. Vecteur comprenant un acide nucléique tel que défini dans la revendication 11.

13. Cellule transfectée, infectée ou transformée avec un acide nucléique selon la revendication 11 ou un vecteur selon la revendication 12.

14. Méthode de production d'une cellule hôte recombinante exprimant un nanocorps tel que défini selon l'une quelconque des revendications 1 à 3, ladite méthode comprenant les étapes consistant à :
(i) introduire *in vitro* ou *ex vivo* un acide nucléique selon la revendication 11 ou un vecteur selon la revendication 12 dans une cellule hôte compétente,
(ii) cultiver *in vitro* ou *ex vivo* la cellule hôte recombinante obtenue et
(iii) éventuellement sélectionner les cellules qui expriment et/ou sécrètent ledit nanocorps.

15. Méthode de production d'un nanocorps tel que défini selon l'une quelconque des revendications 1 à 3, ladite méthode comprenant les étapes consistant à :
(i) cultiver une cellule transfectée ou infectée ou transformée selon la revendication 13, dans des conditions appropriées pour permettre l'expression dudit nanocorps, et
(ii) récupérer le nanocorps exprimé.

## Patentansprüche

1. Nanobody gerichtet gegen VCAM-1 umfassend:
a) die Aminosäuresequenzen (i) YTNSIMYMA (SEQ ID NO: 1) wie CDR1, (ii) AIRFPDDS (SEQ ID NO: 2) wie CDR2 und (iii) RSSPYSFAWNDPSNYNY (SEQ ID NO: 3) wie CDR3; oder
b) die Aminosäuresequenzen (i) FTYSSYYMS (SEQ ID NO: 4) wie CDR1, (ii) GINVDGSN (SEQ ID NO: 5) wie CDR2 und (iii) GSGRDSYDCYSGSWCP (SEQ ID NO: 6) wie CDR3; oder
c) die Aminosäuresequenzen (i) FTFSNYYMT (SEQ ID NO: 7) wie CDR1, (ii) RINSDGS (SEQ ID NO: 8) wie CDR2 und (iii) GKSSV (SEQ ID NO: 9) wie CDR3; oder
d) die Aminosäuresequenzen (i) FTFSSYYMS (SEQ ID NO: 10) wie CDR1, (ii) GINVDGSN (SEQ ID NO: 11) wie CDR2 und (iii) GSGRDSYDCYSGSWCP (SEQ ID NO: 12) wie CDR3;
oder eine konservative, funktionelle Variante des in a), b), c) oder d) definierten Nanobodys umfassend eine konservative Substitution von einer oder zwei Aminosäuren in einer, zwei beziehungsweise drei der Sequenzen SEQ ID NO: 1, SEQ ID NO: 2 und SEQ ID NO: 3 oder SEQ ID NO: 4, SEQ ID NO: 5 und SEQ ID NO: 6 oder SEQ ID NO: 7, SEQ ID NO: 8 und SEQ ID NO: 9 oder SEQ ID NO: 10, SEQ ID NO: 11 und SEQ ID NO: 12.

2. Nanobody gemäß Anspruch 1, wobei der Nanobody eine Aminosäuresequenz ausgewählt aus der Gruppe bestehend aus den Aminosäuresequenzen SEQ ID NO: 13, SEQ ID NO: 14, SEQ ID NO: 15 und SEQ ID NO: 16 umfasst.

3. Nanobody gemäß Anspruch 1 oder 2, wobei der Nanobody aus der Aminosäuresequenz SEQ ID NO: 13 besteht.

4. Nanobody gemäß einem der Ansprüche 1 bis 3, wobei der Nanobody mit einem detektierbaren Marker verbunden ist.

5. Nanobody gemäß Anspruch 4, wobei der detektierbare Marker ein Radioelement ist.

6. Nanobody gemäß einem der Ansprüche 1 bis 5 zur Verwendung als Kontrastmittel in der nicht-invasiven *in vivo* medizinischen Bildgebung.

7. Nanobody gemäß einem der Ansprüche 1 bis 6 zur Verwendung in diagnostischen oder prognostischen Verfahren.

8. Nanobody gemäß einem der Ansprüche 1 bis 3 zur Verwendung als Medikament.

9. Verwendung eines Nanobodys wie in einem der Ansprüche 1 bis 5 definiert zur *in vitro* Detektion von VCAM-1 in einer Probe.

10. Pharmazeutische Zusammensetzung umfassend einen Nanobody wie in einem der Ansprüche 1 bis 3 definiert in Verbindung mit einem pharmazeutisch annehmbaren Trägerstoff.

11. Nukleinsäure umfassend eine Nukleinsequenz, die für den Nanobody wie in einem der Ansprüche 1 bis 3 definiert kodiert.

12. Vektor umfassend eine Nukleinsäure wie in Anspruch 11 definiert.

13. Mit einer Nukleinsäure gemäß Anspruch 11 oder einem Vektor gemäß Anspruch 12 transfizierte, infizierte oder transformierte Zelle.

14. Verfahren zur Herstellung einer rekombinanten Wirtszelle, die einen Nanobody wie in einem der Ansprüche 1 bis 3 definiert exprimiert, wobei das Verfahren die Schritte bestehend aus:
(i) *in vitro* oder *ex vivo* Einführen einer Nukleinsäure gemäß Anspruch 11 oder eines Vektors gemäß Anspruch 12 in eine kompetente Wirtszelle,
(ii) *in vitro* oder *ex vivo* Kultivieren der erhaltenen rekombinanten Wirtszelle und
(iii) wahlweise Selektieren der Zellen, die den Nanobody exprimieren und/oder sekretieren,
umfasst.

15. Verfahren zur Herstellung eines Nanobodys wie in einem der Ansprüche 1 bis 3 definiert, wobei das Verfahren die Schritte bestehend aus:
(i) Kultivieren einer transfizierten oder infizierten oder transformierten Zelle gemäß Anspruch 13 unter Bedingungen, die geeignet sind, die Expression des Nanobodys zu erlauben, und
(ii) Wiedergewinnen des exprimierten Nanobodys
umfasst.

## Claims

1. A nanobody directed against VCAM-1 comprising:
a) the amino acid sequences (i) YTNSIMYMA (SEQ ID NO: 1) as CDR1, (ii) AIRFPDDS (SEQ ID NO: 2) as CDR2 and (iii) RSSPYSFAWNDPSNYNY (SEQ ID NO: 3) as CDR3; or
b) the amino acid sequences (i) FTYSSYYMS (SEQ ID NO: 4) as CDR1, (ii) GINVDGSN (SEQ ID NO: 5) as CDR2 and (iii) GSGRDSYDCYSGSWCP (SEQ ID NO: 6) as CDR3; or
c) the amino acid sequences (i) FTFSNYYMT (SEQ ID NO: 7) as CDR1, (ii) RINSDGS (SEQ ID NO: 8) as CDR2 and (iii) GKSSV (SEQ ID NO: 9) as CDR3 ; or
d) the amino acid sequences (i) FTFSSYYMS (SEQ ID NO: 10) as CDR1, (ii) GINVDGSN (SEQ ID NO: 11) as CDR2 and (iii) GSGRDSYDCYSGSWCP (SEQ ID NO: 12) as CDR3;
or a functionally conservative variant of the nanobody defined in a), b), c) or d) comprising conservative substitution of one or two amino acids in one, two or three of the sequences respectively SEQ ID NO: 1, SEQ ID NO: 2 and SEQ ID NO: 3, or SEQ ID NO: 4, SEQ ID NO: 5 and SEQ ID NO: 6, or SEQ ID NO: 7, SEQ ID NO: 8 and SEQ ID NO: 9, or SEQ ID NO: 10, SEQ ID NO: 11 and SEQ ID NO: 12.

2. The nanobody according to claim 1, said nanobody comprising an amino acid sequence selected from the group consisting of the amino acid sequences SEQ ID NO: 13, SEQ ID NO: 14, SEQ ID NO: 15 and SEQ ID NO: 16.

3. The nanobody according to claim 1 or 2, said nanobody consisting of the amino acid sequence SEQ ID NO: 13.

4. The nanobody according to any of claims 1 to 3, said nanobody being associated with a detectable marker.

5. The nanobody according to claim 4, said detectable marker being a radionuclide.

6. The nanobody according to any of claims 1 to 5 for its use as a contrast agent in non-invasive medical imaging *in vivo.*

7. The nanobody according to any of claims 1 to 6, for its use in diagnostic or prognostic methods.

8. The nanobody according to any of claims 1 to 3 for its use as a drug.

9. The use of a nanobody as defined in any of claims 1 to 5 for *in vitro* detection of VCAM-1 in a sample.

10. A pharmaceutical composition comprising a nanobody as defined in any of claims 1 to 3 in association with a pharmaceutically acceptable carrier.

11. A nucleic acid comprising a nucleic sequence coding for the nanobody as defined in any of claims 1 to 3.

12. A vector comprising a nucleic acid as defined in claim 11.

13. A transfected, infected or transformed cell with a nucleic acid according to claim 11 or a vector according to claim 12.

14. A method for producing a recombinant host cell expressing a nanobody as defined according to any of claims 1 to 3, said method comprising the steps of:
(i) introducing *in vitro* or *ex vivo* nucleic acid according to claim 11 or a vector according to claim 12 into a competent host cell,
(ii) cultivating *in vitro* or *ex vivo* the obtained recombinant host cell and
(iii) optionally selecting the cells which express and/or secrete said nanobody.

15. A method for producing a nanobody as defined according to any of claims 1 to 3, said method comprising the steps of:
(i) cultivating a transfected or infected or transformed cell according to claim 13, under suitable conditions for allowing the expression of said nanobody, and
(ii) recovering the expressed nanobody.


### SEQUENCE LISTING

<110> Université Joseph Fourier
<120> Nanocorps anti-VCAM-1
<130> BET12P2177
<150> FR 11 57478
   <151> 2011-08-23
<160> 36
<170> PatentIn version 3.5
<210> 1
   <211> 9
   <212> PRT
   <213> Artificial
<220>
   <223> CDR1 de cAbVCM1-5
<400> 1
   Tyr Thr Asn Ser Ile Met Tyr Met Ala
   1 5
<210> 2
   <211> 8
   <212> PRT
   <213> Artificial
<220>
   <223> CDR2 de cAbVCAM1-5
<400> 2
   Ala Ile Arg Phe Pro Asp Asp Ser
   1 5
<210> 3
   <211> 17
   <212> PRT
   <213> Artificial
<220>
   <223> CDR3 de cAbVCAM1-5
<400> 3
   Arg Ser Ser Pro Tyr Ser Phe Ala Trp Asn Asp Pro Ser Asn Tyr Asn
   1 5 10 15
   Tyr
<210> 4
   <211> 9
   <212> PRT
   <213> Artificial
<220>
   <223> CDR1 de cAbVCAM1-3
<400> 4
   Phe Thr Tyr Ser Ser Tyr Tyr Met Ser
   1 5
<210> 5
   <211> 8
   <212> PRT
   <213> Artificial
<220>
   <223> CDR2 de cAbVCAM1-3
<400> 5
   Gly Ile Asn Val Asp Gly Ser Asn
   1 5
<210> 6
   <211> 16
   <212> PRT
   <213> Artificial
<220>
   <223> CDR3 de cAbVCAM1-3
<400> 6
   Gly Ser Gly Arg Asp Ser Tyr Asp Cys Tyr Ser Gly Ser Trp Cys Pro
   1 5 10 15
<210> 7
   <211> 9
   <212> PRT
   <213> Artificial
<220>
   <223> CDR1 de cAbVCAM1-8
<400> 7
   Phe Thr Phe Ser Asn Tyr Tyr Met Thr
   1 5
<210> 8
   <211> 7
   <212> PRT
   <213> Artificial
<220>
   <223> CDR2 de cAbVCAM1-8
<400> 8
   Arg Ile Asn Ser Asp Gly Ser
   1 5
<210> 9
   <211> 5
   <212> PRT
   <213> Artificial
<220>
   <223> CDR3 de cAbVCAM1-8
<400> 9
   Gly Lys Ser Ser Val
   1 5
<210> 10
   <211> 9
   <212> PRT
   <213> Artificial
<220>
   <223> CDR1 de cAbVCAM1-9
<400> 10
   Phe Thr Phe Ser Ser Tyr Tyr Met Ser
   1               5
<210> 11
   <211> 8
   <212> PRT
   <213> Artificial
<220>
   <223> CDR2 de cAbVCAM1-9
<400> 11
   Gly Ile Asn Val Asp Gly Ser Asn
   1               5
<210> 12
   <211> 16
   <212> PRT
   <213> Artificial
<220>
   <223> CDR3 de cAbVCAM1-9
<400> 12
   Gly Ser Gly Arg Asp Ser Tyr Asp Cys Tyr Ser Gly Ser Trp Cys Pro
   1               5                   10                  15
<210> 13
   <211> 126
   <212> PRT
   <213> Artificial
<220>
   <223> cAbVCAM1-5
<400> 13
   Gln Val Gln Leu Gln Glu Ser Gly Gly Gly Ser Val Gln Thr Gly Gly
   1 5 10 15
   Ser Leu Arg Leu Ser Cys Ala Ala Ser Gly Tyr Thr Asn Ser Ile Met
   20 25 30
   Tyr Met Ala Trp Phe Arg Gln Ala Pro Gly Lys Lys Arg Glu Gly Val
   35 40 45
   Ala Ala Ile Arg Phe Pro Asp Asp Ser Ala Tyr Tyr Ala Gly Ser Val
   50 55 60
   Lys Gly Arg Phe Thr Ile Ser His Asp Asn Ala Lys Asn Thr Val Tyr
   65 70 75 80
   Leu Gln Met Asn Asn Leu Asn Pro Glu Asp Thr Ala Met Tyr Tyr Cys
   85 90 95
   Ala Ala Arg Ser Ser Pro Tyr Ser Phe Ala Trp Asn Asp Pro Ser Asn
   100 105 110
   Tyr Asn Tyr Trp Gly Gln Gly Thr Gln Val Thr Val Ser Ser
   115 120 125
<210> 14
   <211> 125
   <212> PRT
   <213> Artificial
<220>
   <223> cAbVCAM1-3
<400> 14
   Gln Val Gln Leu Gln Glu Ser Gly Gly Gly Ser Val Gln Ala Gly Gly
   1               5                   10                  15
   Ser Leu Arg Leu Ser Cys Thr Ala Ser Gly Phe Thr Tyr Ser Ser Tyr
               20                  25                  30
   Tyr Met Ser Trp Val Arg Gln Ala Pro Gly Lys Gly Leu Glu Trp Val
           35                  40                  45
   Ser Gly Ile Asn Val Asp Gly Ser Asn Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Ser Glu Asp Thr Ala Leu Tyr Tyr Cys
                   85                  90                  95
   Ala Thr Gly Ser Gly Arg Asp Ser Tyr Asp Cys Tyr Ser Gly Ser Trp
               100                 105                 110
   Cys Pro Lys Gly Gln Gly Thr Gln Val Thr Val Ser Ser
           115                 120                 125
<210> 15
   <211> 113
   <212> PRT
   <213> Artificial
<220>
   <223> cAbVCAM1-8
<400> 15
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Tyr Met Thr Trp Val Arg Arg Ala Pro Gly Lys Gly Leu Glu Trp Val
           35                  40                  45
   Ser Arg Ile Asn Ser Asp Gly Ser Thr Leu Tyr Leu Pro Ser Val Lys
       50                  55                  60
   Gly Arg Phe Thr Ile Ser Arg Asp Asn Ala Lys Asn Thr Leu Tyr Leu
   65                  70                  75                  80
   Gln Met Asn Ser Leu Lys Ser Glu Asp Thr Gly Trp Tyr Tyr Cys Val
                   85                  90                  95
   Glu Gly Lys Ser Ser Val Arg Gly Gln Gly Thr Gln Val Thr Val Ser
               100                 105                 110
   Ser
<210> 16
   <211> 125
   <212> PRT
   <213> Artificial
<220>
   <223> cAbVCAM1-9
<400> 16
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Tyr Met Ser Trp Val Arg Gln Ala Pro Gly Lys Gly Leu Glu Trp Val
           35                  40                  45
   Ser Gly Ile Asn Val Asp Gly Ser Asn Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Ser Glu Asp Thr Ala Leu Tyr Tyr Cys
                   85                  90                  95
   Ala Thr Gly Ser Gly Arg Asp Ser Tyr Asp Cys Tyr Ser Gly Ser Trp
               100                 105                 110
   Cys Pro Lys Gly Gln Gly Thr Gln Val Thr Val Ser Ser
           115                 120                 125
<210> 17
   <211> 26
   <212> PRT
   <213> Artificial
<220>
   <223> FR1 de cAbVCAM1-5
<400> 17
   Gln Val Gln Leu Gln Glu Ser Gly Gly Gly Ser Val Gln Thr Gly Gly
   1               5                   10                  15
   Ser Leu Arg Leu Ser Cys Ala Ala Ser Gly
               20                  25
<210> 18
   <211> 14
   <212> PRT
   <213> Artificial
<220>
   <223> FR2 de cAbVCAM1-5
<400> 18
   Trp Phe Arg Gln Ala Pro Gly Lys Lys Arg Glu Gly Val Ala
   1               5                   10
<210> 19
   <211> 41
   <212> PRT
   <213> Artificial
<220>
   <223> FR3 de cAbVCAM1-5
<400> 19
   Ala Tyr Tyr Ala Gly Ser Val Lys Gly Arg Phe Thr Ile Ser His Asp
   1               5                   10                  15
   Asn Ala Lys Asn Thr Val Tyr Leu Gln Met Asn Asn Leu Asn Pro Glu
               20                  25                  30
   Asp Thr Ala Met Tyr Tyr Cys Ala Ala
           35                  40
<210> 20
   <211> 11
   <212> PRT
   <213> Artificial
<220>
   <223> FR4 de cAbVCAM1-5
<400> 20
   Trp Gly Gln Gly Thr Gln Val Thr Val Ser Ser
   1               5                   10
<210> 21
   <211> 26
   <212> PRT
   <213> Artificial
<220>
   <223> FR1 de cAbVCAM1-3
<400> 21
   Gln Val Gln Leu Gln Glu Ser Gly Gly Gly Ser Val Gln Ala Gly Gly
   1               5                   10                  15
   Ser Leu Arg Leu Ser Cys Thr Ala Ser Gly
               20                  25
<210> 22
   <211> 14
   <212> PRT
   <213> Artificial
<220>
   <223> FR2 de cAbVCAM1-3
<400> 22
   Trp Val Arg Gln Ala Pro Gly Lys Gly Leu Glu Trp Val Ser
   1 5 10
<210> 23
   <211> 41
   <212> PRT
   <213> Artificial
<220>
   <223> FR3 de cAbVCAM1-3
<400> 23
   Thr Tyr Tyr Ala Asp Ser Val Lys Gly Arg Phe Thr Ile Ser Arg Asp
   1 5 10 15
   Asn Ala Lys Asn Thr Leu Tyr Leu Gln Met Asn Ser Leu Lys Ser Glu
   20 25 30
   Asp Thr Ala Leu Tyr Tyr Cys Ala Thr
   35 40
<210> 24
   <211> 11
   <212> PRT
   <213> Artificial
<220>
   <223> FR4 de cAbVCAM1-3
<400> 24
   Lys Gly Gln Gly Thr Gln Val Thr Val Ser Ser
   1 5 10
<210> 25
   <211> 26
   <212> PRT
   <213> Artificial
<220>
   <223> FR1 de cAbVCAM1-8
<400> 25
   Gln Val Gln Leu Gln Glu Ser Gly Gly Gly Leu Val Gln Pro Gly Gly
   1 5 10 15
   Ser Leu Arg Leu Ser Cys Ala Ala Ser Gly
   20 25
<210> 26
   <211> 14
   <212> PRT
   <213> Artificial
<220>
   <223> FR2 de cAbVCAM1-8
<400> 26
   Trp Val Arg Arg Ala Pro Gly Lys Gly Leu Glu Trp Val Ser
   1 5 10
<210> 27
   <211> 41
   <212> PRT
   <213> Artificial
<220>
   <223> FR3 de cAbVCAM1-8
<400> 27
   Thr Leu Tyr Leu Pro Ser Val Lys Gly Arg Phe Thr Ile Ser Arg Asp
   1 5 10 15
   Asn Ala Lys Asn Thr Leu Tyr Leu Gln Met Asn Ser Leu Lys Ser Glu
   20 25 30
   Asp Thr Gly Trp Tyr Tyr Cys Val Glu
   35 40
<210> 28
   <211> 11
   <212> PRT
   <213> Artificial
<220>
   <223> FR4 de cAbVCAM1-8
<400> 28
   Arg Gly Gln Gly Thr Gln Val Thr Val Ser Ser
   1 5 10
<210> 29
   <211> 26
   <212> PRT
   <213> Artificial
<220>
   <223> FR1 de cAbVCAM1-9
<400> 29
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly
               20                  25
<210> 30
   <211> 14
   <212> PRT
   <213> Artificial
<220>
   <223> FR2 de cAbVCAM1-9
<400> 30
   Trp Val Arg Gln Ala Pro Gly Lys Gly Leu Glu Trp Val Ser
   1               5                   10
<210> 31
   <211> 41
   <212> PRT
   <213> Artificial
<220>
   <223> FR3 de cAbVCAM1-9
<400> 31
   Thr Tyr Tyr Ala Asp Ser Val Lys Gly Arg Phe Thr Ile Ser Arg Asp
   1               5                   10                  15
   Asn Ala Lys Asn Thr Leu Tyr Leu Gln Met Asn Ser Leu Lys Ser Glu
               20                  25                  30
   Asp Thr Ala Leu Tyr Tyr Cys Ala Thr
           35                  40
<210> 32
   <211> 11
   <212> PRT
   <213> Artificial
<220>
   <223> FR4 de cAbVCAM1-9
<400> 32
   Lys Gly Gln Gly Thr Gln Val Thr Val Ser Ser
   1               5                   10
<210> 33
   <211> 132
   <212> PRT
   <213> Artificial
<220>
   <223> cAbVCAM1-5 6His
<400> 33
   Gln Val Gln Leu Gln Glu Ser Gly Gly Gly Ser Val Gln Thr Gly Gly
   1               5                   10                  15
   Ser Leu Arg Leu Ser Cys Ala Ala Ser Gly Tyr Thr Asn Ser Ile Met
               20                  25                  30
   Tyr Met Ala Trp Phe Arg Gln Ala Pro Gly Lys Lys Arg Glu Gly Val
           35                  40                  45
   Ala Ala Ile Arg Phe Pro Asp Asp Ser Ala Tyr Tyr Ala Gly Ser Val
       50                  55                  60
   Lys Gly Arg Phe Thr Ile Ser His Asp Asn Ala Lys Asn Thr Val Tyr
   65                  70                  75                  80
   Leu Gln Met Asn Asn Leu Asn Pro Glu Asp Thr Ala Met Tyr Tyr Cys
                   85                  90                  95
   Ala Ala Arg Ser Ser Pro Tyr Ser Phe Ala Trp Asn Asp Pro Ser Asn
               100                 105                 110
   Tyr Asn Tyr Trp Gly Gln Gly Thr Gln Val Thr Val Ser Ser His His
           115                 120                 125
   His His His His
       130
<210> 34
   <211> 131
   <212> PRT
   <213> Artificial
<220>
   <223> cAbVCAM1-3 6His
<400> 34
   Gln Val Gln Leu Gln Glu Ser Gly Gly Gly Ser Val Gln Ala Gly Gly
   1               5                   10                  15
   Ser Leu Arg Leu Ser Cys Thr Ala Ser Gly Phe Thr Tyr Ser Ser Tyr
               20                  25                  30
   Tyr Met Ser Trp Val Arg Gln Ala Pro Gly Lys Gly Leu Glu Trp Val
           35                  40                  45
   Ser Gly Ile Asn Val Asp Gly Ser Asn Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Ser Glu Asp Thr Ala Leu Tyr Tyr Cys
                   85                  90                  95
   Ala Thr Gly Ser Gly Arg Asp Ser Tyr Asp Cys Tyr Ser Gly Ser Trp
               100                 105                 110
   Cys Pro Lys Gly Gln Gly Thr Gln Val Thr Val Ser Ser His His His
           115                 120                 125
   His His His
       130
<210> 35
   <211> 119
   <212> PRT
   <213> Artificial
<220>
   <223> 6His
<400> 35
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Tyr Met Thr Trp Val Arg Arg Ala Pro Gly Lys Gly Leu Glu Trp Val
           35                  40                  45
   Ser Arg Ile Asn Ser Asp Gly Ser Thr Leu Tyr Leu Pro Ser Val Lys
       50                  55                  60
   Gly Arg Phe Thr Ile Ser Arg Asp Asn Ala Lys Asn Thr Leu Tyr Leu
   65                  70                  75                  80
   Gln Met Asn Ser Leu Lys Ser Glu Asp Thr Gly Trp Tyr Tyr Cys Val
                   85                  90                  95
   Glu Gly Lys Ser Ser Val Arg Gly Gln Gly Thr Gln Val Thr Val Ser
               100                 105                 110
   Ser His His His His His His
           115
<210> 36
   <211> 131
   <212> PRT
   <213> Artificial
<220>
   <223> cAbVCAM1-9 6His
<400> 36
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Tyr Met Ser Trp Val Arg Gln Ala Pro Gly Lys Gly Leu Glu Trp Val
                   35                  40                  45
   Ser Gly Ile Asn Val Asp Gly Ser Asn Thr Tyr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Ser Glu Asp Thr Ala Leu Tyr Tyr Cys
                   85                  90                  95
   Ala Thr Gly Ser Gly Arg Asp Ser Tyr Asp Cys Tyr Ser Gly Ser Trp
               100                 105                 110
   Cys Pro Lys Gly Gln Gly Thr Gln Val Thr Val Ser Ser His His His
           115                 120                 125
   His His His
       130